(19) **Europäisches Patentamt**

**European Patent Office**

**Office européen des brevets**

(11) **EP 1 362 510 B1**

(12) **EUROPEAN PATENT SPECIFICATION**

(45) Date of publication and mention
of the grant of the patent:
**01.03.2006 Bulletin 2006/09**

(51) Int Cl.:
*A01K 67/027* (2006.01)　　*C12N 5/10* (2006.01)

(21) Application number: **03010880.7**

(22) Date of filing: **15.05.2003**

(54) **A mouse model for hepatocellular carcinoma by targeted integration of hepatitis B virus genes**

Mausmodell für Leberzellkarzinom durch zielgerichtete Integration von Hepatitis B Virus Genen

Modèle de souris pour le carcinome hépatocellulaire par intégration ciblée des gènes du virus de l'hépatite B

(84) Designated Contracting States:
**AT BE BG CH CY CZ DE DK EE ES FI FR GB GR HU IE IT LI LU MC NL PT RO SE SI SK TR**

(30) Priority: **17.05.2002 CN 02117467**

(43) Date of publication of application:
**19.11.2003 Bulletin 2003/47**

(73) Proprietor: **Beijing Institute of Biotechnology Beijing 100071 (CN)**

(72) Inventors:
 • **Yang, Xiao,**
  c/o Beijing Inst. of Biotechnology
  Beijing 100071 (CN)
 • **Wang, Youliang,**
  c/o Beijing Inst. of Biotechnology
  Beijing 100071 (CN)
 • **Lu, Yaxin,**
  c/o Beijing Inst. of Biotechnology
  Beijing 100071 (CN)
 • **Huang, Cuifen,**
  c/o Beijing Inst. of Biotechnology
  Beijing 100071 (CN)
 • **Cui, Fang,**
  c/o Beijing Inst. of Biotechnology
  Beijing 100071 (CN)

(74) Representative: **Gervasi, Gemma et al Notarbartolo & Gervasi S.p.A.,
Corso di Porta Vittoria, 9
20122 Milano (IT)**

(56) References cited:
　**WO-A-98/10059**　　　**US-B1- 6 274 788**

 • **DATABASE WPI Section Ch, Week 199712 Derwent Publications Ltd., London, GB; Class B04, AN 1997-126423 XP002255100 & JP 09 009965 A (ZH KAGAKU & KESSEI RYOHO KENKYUSHO), 14 January 1997 (1997-01-14)**
 • **DENG CHUXIA ET AL: "Mice Lacking p21-CIP1/WAF1 Undergo Normal Development, but Are Defective in G1 Checkpoint Control." CELL, vol. 82, no. 4, 1995, pages 675-684, XP001160829 ISSN: 0092-8674**
 • **CHISARI F V ET AL: "MOLECULAR PATHOGENESIS OF HEPATOCELLULAR CARCINOMA IN HEPATITIS B VIRUS TRANSGENIC MICE" CELL, vol. 59, no. 6, 1989, pages 1145-1156, XP001160834 ISSN: 0092-8674**
 • **TERRADILLOS OLIVIER ET AL: "The hepatitis B virus X gene potentiates c-myc-induced liver oncogenesis in transgenic mice." ONCOGENE, vol. 14, no. 4, 1997, pages 395-404, XP002255068 ISSN: 0950-9232**
 • **QIAO LIANG ET AL: "Hepatitis B virus X protein increases expression of p21Cip-1/WAF1/MDA6 and p27Kip-1 in primary mouse hepatocytes, leading to reduced cell cycle progression." HEPATOLOGY, vol. 34, no. 5, November 2001 (2001-11), pages 906-917, XP008022221 ISSN: 0270-9139**

**Description**

**TECHNIQUE FIELD OF THE INVENTION**

The invention relates to a transgenic animal model and a method for establishing such model. Particularly, the invention relates to a method for integrating an exogenous gene of interest into an animal genome by embryonic stem (ES) cells culture and homologous recombination to generate an animal model in which the exogenous gene is expressed stably, as well as to animal model obtained by the method.

**BACKGROUND OF THE INVENTION**

Hepatocellular carcinoma (HCC) is one of the leading malignancies worldwide, which especially distributes in Asia and Pacific regions including China. More than 1 million people develop into HCC each year[1], wherein five year survival rate is lower than 5%. A number of etiological factors, particularly hepatitis B virus (HBV) infection, are involved in the occurrence and progression of HCC. HBV belongs to DNA virus, which has a full length approximately 3.2kb containing a circular, partially double stranded DNA and replicating via an RNA intermediate. HBV can randomly integrate into host genome, and the host integrating HBV is referred to as carrier, who is in a state of chronic infection without symptom, but with the expression of hepatitis B surface antigen (*HBsAg*). It has been estimated that at least 350 million people worldwide are HBV chronic carriers[2], more than 75% being in Asia and Western Pacific[3]. Among these carriers, there are at least about 20% showing various degrees of chronic hepatitis and cirrhosis, and parts of them may develop into primary hepatocellular carcinoma. Clinical and epidemiological studies have showed that the risk of being afflicted with HCC reaches as high as 40 to 50% among HBV chronic carriers. For hepatocellular carcinoma, the best clinical approach is hepatocarcinoma resection at early stage, thus early stage diagnosis appears to be significantly important. However, no molecular marker in serum or liver that can be conveniently used to diagnose carriers who possibly develop into HCC with the least delay has been found yet. Further studies on pathology and molecular mechanisms responsible for HBV-induced HCC have great meaning for early stage diagnosis of such hepatocarcinoma.

The induction and progression of hepatocellular carcinoma is a complex progress involving multiple factors, which relates to abnormal gene expression or mutation and interaction between them. After HBV infection, the interactions between HBV gene expression products and hepatocyte proteins or nucleic acids can interfere with normal gene expression and signal transduction, thus may trigger abnormal expression of proto-oncogenes, or functionally inactivate tumor suppressor genes, leading to abnormal growth and differentiation of hepatocyte, and finally to hepatocellular carcinoma. Up to now, however, the targets of HBV are unclear. Since HBV commonly infects neither cultured cells nor ordinary experimental animals (chimpanzee being the onlysensitive experimental animal), this has limited the research on HBV pathogenesis to some degrees. Transgenic animal technique provides new approach to investigate HBV pathogenesis. Chisari[4] and Babinet[5] et al. successfully established a number of HBV transgenic mouse models using transgenic technique in 1985, since then studies on such mouse models havegreatly promoted the understanding of pathology and molecular mechanisms of HBV-induced hepatocellular carcinoma. However, such transgenic mice are developed by traditional transgenic technique, and the exogenous genes in them are randomly integrated into chromosomes, often resulting in phenotype difference and to some degrees making phenotype analysis difficult. Thus, final conclusions regarding molecular mechanisms of HBV-induced HCC haven't been made yet. Although domestic reports on transgenic mouse integrating entire HBV genome have been published, there hasn't report on successful transgenic mouse model for hepatocarcinoma up to now.

Since late eighties, on the basis of gene targeting technique, gene knock-in technique has been developed, which uses the technologies of homologous recombination and mouse ES cell culture to integrate an exogenous gene into a specific site on mouse genome, obviating blindness of traditional transgenic technology. Deng[6] found that mice developed normally and no abnormal phenotype existed in the liver after its *p21* gene was removed. Moreover, the implementation of human genome mapping marks the beginning of a post-genomic era in life science, and the research on proteomics is becoming an important tool in studying functional genomics.

**DESCRIPTION OF THE INVENTION**

The invention relates to a method for establishing an non-human animal model comprising:

a) inserting an exogenous gene of interest into a suitable vector carrying sequences homologous to a target site, to construct a recombinant targeting vector;
b) transfecting ES cells of a studied non-human animal using said targeting vector from step a), and screening targeted ES cells which have integrated the exogenous gene into a specific site;
c) injecting targeted ES cells obtained from step b) into said non-human animal's blastulas and culturing the blastulas in vitro, so that embryos containing targeted ES cells are generated;
d) implanting embryos generated from step c) into said non-human animal's uterus, thereby progeny expressing the exogenous gene stably are developed.

wherein said exogenous gene of interest is a HBV or a HCV gene and said exogenous gene of interest is inserted into the p21 locus of said non-human animal.

## BRIEF DESCRIPTION OF THE DRAWINGS

Figures.1(a)-(d) illustrates the construction of a targeting vector, the vector is used to integrate *HBsAg* gene to a specific site on transgenic mouse genome.
Figures.2(a)-(d) illustrates the construction of a targeting vector, the vector is used to integrate HBV X gene to a specific site on transgenic mouse genome.
Figure.3 shows Southern blot screening of embryo stem cells from transgenic mice containing targeted integration of *HBsAg*.
Figure.4 shows PCR identification of transgenic mice containing targeted integration of *HBsAg*.
Figure.5 shows Southern blot identification of transgenic mice containing targeted integration of *HBsAg*.
Figure.6 shows Northern blot analysis of transgenic mice containing targeted integration of *HBsAg*.
Figure.7 shows hematoxylin and eosin staining of liver tissue from a 15 month old mouse.
Figure.8 shows PCNA staining of liver tissue from a 15 month old mouse.
Figure.9 shows the testing of serum $\alpha\alpha$-fetal protein in 2 month old mice.

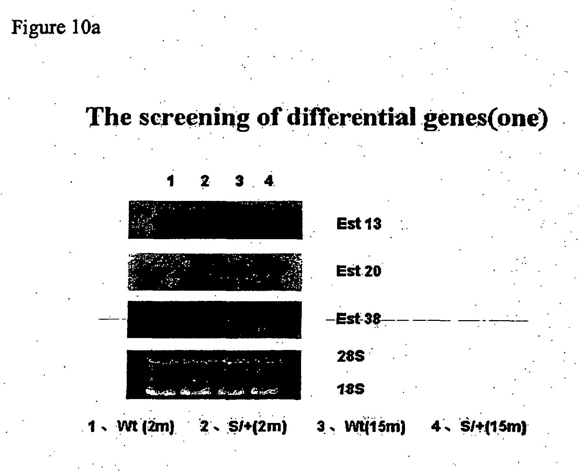

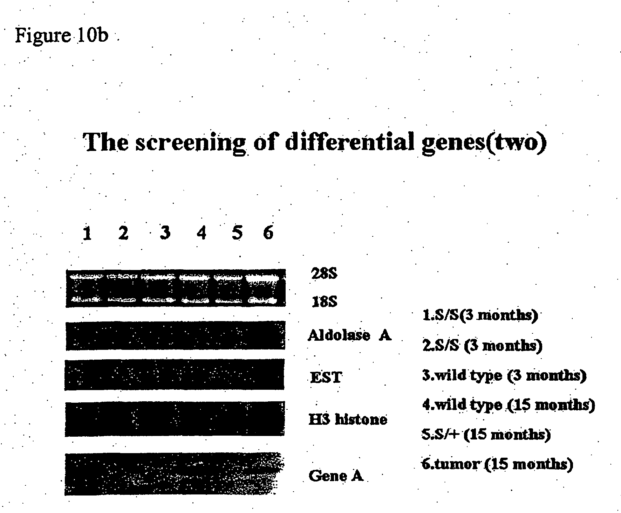

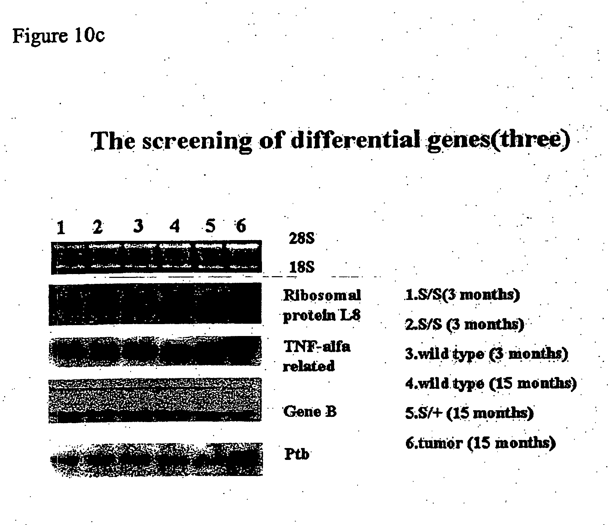

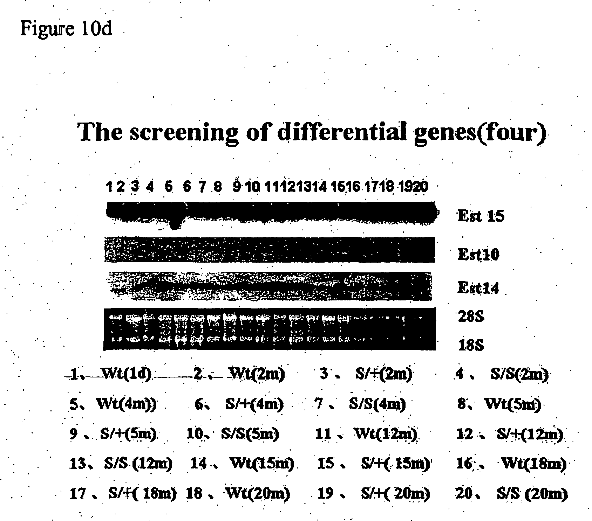

Figures.10 (a)-(d) show Northern blot analysis of genes expressed differentially.

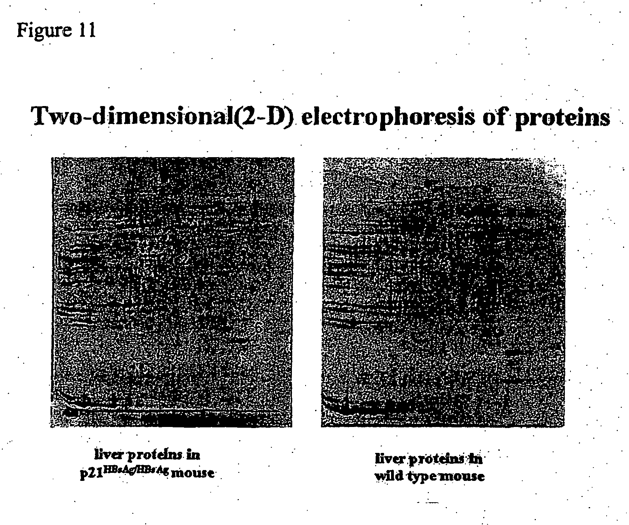

Figure.11 shows the gel scanning image following two-dimensional(2-D) electrophoresis of liver proteins.

In the first aspect, the present invention relates to the construction of targeting transgenic vector, which comprises the following steps:

pLoxpneo[7] was used as initial targeting vector; genomic DNA segment approximately 8kb flanking the second exon of *p21* gene was used as homologous sequence of targeting vector. An exogenous gene (for example viral gene from HBV, HCV, HIV and the like, such as *HBsAg, HBX* et al) was inserted into the second exon of *p21* gene. Following said elements being assembled into targeting transgenic vector, the vector comprises: 1) homologous sequences of target site, wherein the said target site may be *p21* locus or other loci transcribed and expressed in hepatocyte; 2) an exogenous gene, a positive selection marker (*neo*), and a negative selection marker (*tk*). The exogenous gene may be entire HBV genome, individual HBV gene, or may be entire genome or individual gene of other etiologic microorganisms. The positive selection marker may be neo gene or other positive selection markers, such as hygromycin B phosphotransferase (*hph*), xanthine/guanine phosphotransferase (*gpt*), hypoxanthine phosphoribosyl transferase (*hprt*) and the like. The negative selection marker may be tk gene or other negative selection markers, such as herpes simplex virus thymidine kinase (HSVtk), diphtherotoxin (DT), thymidine kinase and the like.

In the second aspect, the present invention relates to the preparation of targeted ES cell, using the following protocol:

The said targeting vector was linearized, then transfected into ES cells by electroporation. Following mixation of transfected ES cells with fresh cell culture medium, these ES cells were distributed to plates where feeder cells had grown to confluency. 24 hours later, the medium was changed into selective medium containing G418 and gancyclovir for screening ES cells, since then changing fresh selective medium daily. After seven days of transfection, cell clones were screened. Specifically speaking, ES clones were dispersed as single cell suspentions, then divided single cell suspension from each ES clone into two equal aliquots and replated onto two 96-well plates with feeder cells respectively. For the two plates, each clone's position and order were the same. Following alteration of the medium into fresh ES cell medium daily and culturing for 2 to 3 days, one plate was frozen, the other one was trypsinized and ES clones in it were transferred to 24-well plates, cultured in feeder cell medium until the medium color turned yellow. Lysis buffer was added to each well, then lysed cell suspension was transferred to Eppendorf tube. After extracting from ES cells and completely dissolving in TE solution, genomic DNA was digested with EcoRI and BglII respectively, and the resultant digests were subject to Southern blot using probes positioned outside 5' end of targeting vector. As regards digestion with Bgl II, a positive band approximately 8 kb should appear for wild type p21 allele, whereas this positive band would shift to 9 kb for targeted p21 allele, the latter results from the deletion of an original Bgl II site and the introduction of another Bgl II site via neo gene after homologous recombination. As regards digestion with EcoRI, a positive band approximately 7.7 kb should appear for wild type p21 allele, whereas this positive band would shift to 22 kb for targeted p21 allele, the latter results from the deletion of an original EcoRI site after homologous recombination. ES cells with the positive band position being altered were ones containing the exogenous gene.

In the third aspect, the present invention relates to a method for the preparation of transgenic animals containing targeted ES cells, which is described as follows:

Firstly, unexcited 4-5 week old female C57BL/6J mice were intraperitoneally injected 5IU gonadotropin. Within 48 hours of the last gonadotropin injection, these mice were intraperitoneally injected 5IU human chorion-gonadotropin to make them superovulate, then transferred to cages with stud male mice and mated with them. 3.5 days later, blastulas in uteri were collected. Meanwhile, ES cells to be injected were thawed several days before, and the medium was changed into fresh ES medium in the morning of the day for injection. 1-2 hours later, the cells were treated with trypsin, and the resultant single cells were stored as suspension in Brinster's BMOC-3 medium, then sucked 12-15 small and round ES cells by injection pipette, pushed injection pump under the microscope to make ES cells enter into blastocoele in sequence. Then the injected blastulas were cultured in droplet supplemented with Brinster's BMOC-3 medium, thereby embryos to be implanted could be obtained. Secondly, after weighted, pseudopregnant female mice were intraperitoneally injected anesthetic and dissected. The embryos to be implanted were blown slowly using a transfer pipette into their uteri under the dissecting microscope, afterwards their uteri and mesenterys were sent back to abdominal cavities and incisions were closed.

If the above operation was successful, pups would be born 17 days later. It could be deduced if highly chimeric mice had been obtained from their coat colors several days later. When highly chimeric mice were selected to mate with C57BL/6J mice, transgenic offspring with pure brown coat would generate.

In the fourth aspect, the present invention relates to analysis of phenotype of transgenic mouse containing *HBsAg* gene, which is described as follows:

About 0.5cm piece of tail tip was cut from 15 day old mouse and placed in an Eppendorf tube, to which lysis buffer was added to make cells lyse, then highly concentrated salts were added to precipitate proteins. After supernatant containing DNA was collected by centrifugation and precipitated by ethanol, genomic DNA could be obtained. The DNA was redissolved in TE solution for later use.

PCR was performed to identify offspring mice with different genotype by using genomic DNA as template and pairs of primers. Primer 1(5'-TCTTCTGTTTCAGCCACAGGC-3') and primer 2(5'-TGTCAGGCTGGTCTG CCTCC-3') were used to identify wild type p21 allele, and a 436 bp band could be amplified from wild type and heterozygous mice. Primer 3(5'-ATTTTCCAGGGATCTGACTC-3') and primer 4(5'-CCAGACTGC CTTGGGAAAAGC-3') on neo gene were used to identify targeted allele containing *neo* gene. Primer 5(5'-GGAC CCTGCACCGAACATGG-3') and primer 6 (5'-GGAATAGCCCCAACGTT TGG-3') were used to identify HBsAg gene.

Mouse tail genomic DNA was digested with EcoRI and BgIII respectively, and the resultant digests were subject to Southern blot using probe a positioned outside 5' end of targeting vector. As regards digestion with Bgl II, a positive band approximately 8 kb should appear for wild type p21 allele, whereas this positive band would shift to approximately 9 kb for targeted p21 allele, the latter resulted from the deletion of an original Bgl II site and the introduction of another Bgl II site via neo gene after homologous recombination. As regards digestion with EcoRI, a positive band approximately 7.7 kb should appear for wild type p21 allele, whereas this positive band containing HBsAg gene would shift to approximately 22 kb for targeted p21 allele, the latter resulted from the deletion of an original EcoRI site after homologous recombination.

Total RNA was extracted from mouse tissue using Trizol agent, then Northern blot analysis was performed. Briefly, 20μg of total RNA was taken to perform formaldehyde denatured agarose gel electrophoresis, then the RNA was transferred from gel to nitrocellulose membrane by capillary transfer, hybridized with probes derived from *HBsAg* gene. Results showed that *HBsAg* gene was expressed in the liver and spleen of transgenic mouse.

The expression of *HBsAg* gene was assayed using one-step HBsAg ELISA kit. Both mouse serum and liver homogenate were prepared, however, for transgenic mice, it was found that HBsAg only existed in liver homogenate, not being secreted into serum.

The growth and development of heterozygous and homozygous transgenic mice were normal. In the liver, lymphocyte infiltration and steatosis could be observed one year later; proliferating cell nuclear antigen (PCNA) staining showed that hepatocytes were in a state of hyperproliferation. 15 month old heterozygous mice began developing toward the well-differendatd hepatocellular carcinoma, and 75 % transgenic male mice over 17 months of age would progress into the condition. Obvious apoptosis was not observed.

In the fifth aspect, the present invention relates to the use of the transgenic mouse as HBV gene induced hepatocellular carcinoma model mouse, which comprises the following studies:

Firstly, total RNA was extract from liver of wild type or HBV transgenic homozygous mouse at different months of age, and gene expression patterns were analyzed by Northern blot to find genes which might be involved in the occurrence of hepatocellular carcinoma, so as to make a preliminary studies on the molecular mechanisms of the disease occurrence. Secondly, proteins were extract from serum or the liver of wild type or HBV transgenic homozygous mouse at different months of age, then subject to two-dimensional(2-D) gel electrophoresis. Comparative analysis was performed to find differentially-expressed proteins, then these proteins were identified through analysis of peptide mass fingerprint profile, in order to make intensive studies on the early events and molecular mechanisms of the induction of hepatocellular carcinoma, screen target molecules for diagnosis or treatment of hepatocellular carcinoma. Finally, drugs were administrated to mice bearing HBV gene-induced hepatocellular carcinoma and wild type mice at different months of age, by a route of intravenous injection, intraperitoneal injection, or oral administration. Tail venous bloods were collected and hepatocellular carcinoma-associated early molecules that had found from previous research were monitored, so as to evaluate therapeutic scheme and drug efficiency. Histopathologic analysis of the liver from mice over 18 month old were carried out, and the efficiency of therapeutic drug was also evaluated.

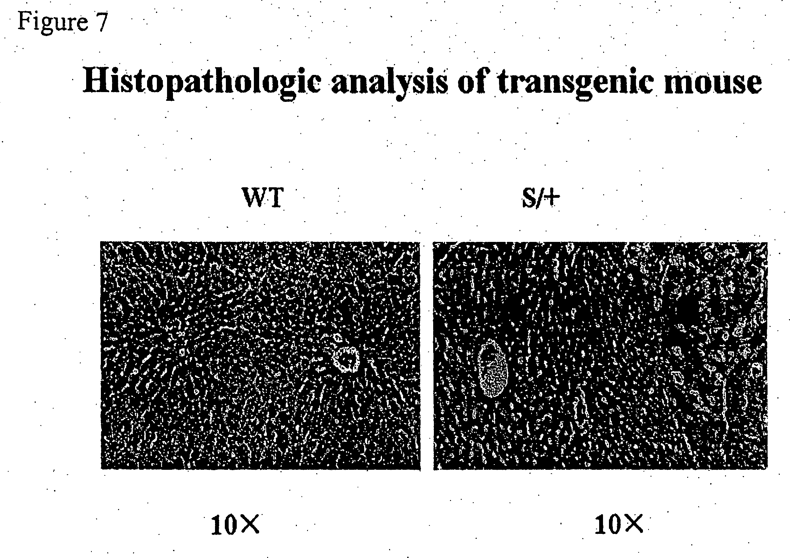

Taking advantage of gene knock-in method, the inventors have integrated hepatitis B surface antigen (*HBsAg*) gene into specific site on mouse ES cell genome by means of homologous recombination and ES cell culture. Transgenic mice expressing *HBsAg* gene stably were obtained through microinjection and embryo implantation. Such transgenic mice expressed hepatitis B surface antigen in the liver, both heterozygous mice and homozygous ones developed normally. In the liver, lymphocyte infiltration and steatosis could be observed one year later; proliferating cell nuclear antigen (PCNA) staining showed that hepatocytes were in a state of hyperproliferation. 15 month old heterozygous mice began developing toward the well-differentiated hepatocellular carcinoma (figure 7), and 75% transgenic male mice over 17 months of age would progress into the condition. Obvious apoptosis was not observed. Such HBV gene induced hepatocellular carcinoma model mouse can be used to make intensive studies on the early events and molecular mechanisms of the induction of hepatocellular carcinoma, to screen target molecules for early stage diagnosis or treatment of hepatocellular carcinoma, and to evaluate therapeutic scheme and efficiency of Chinese traditional medicine and western medicine.

The invention is further illustrated in details by the following examples, and these examples are not to be construed as a limitation on the scope of the invention.

Example 1

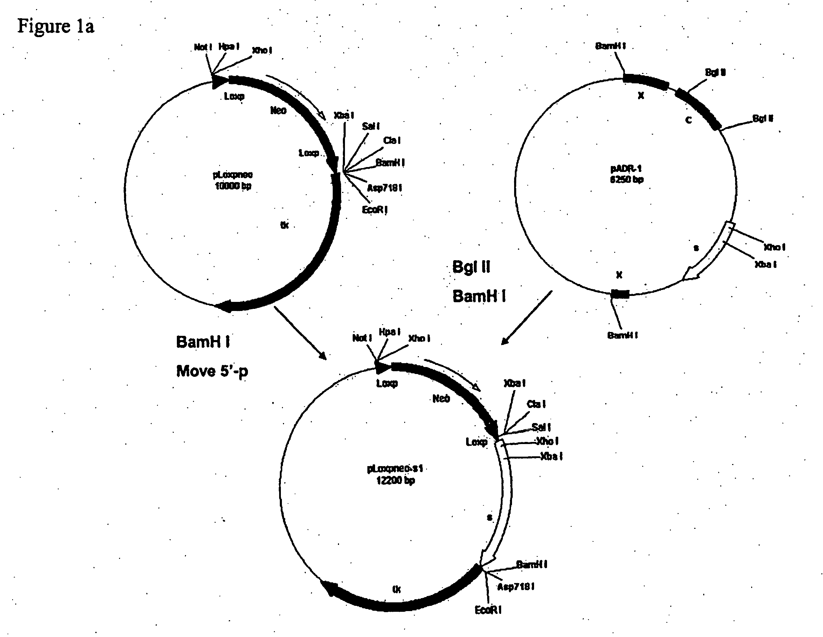

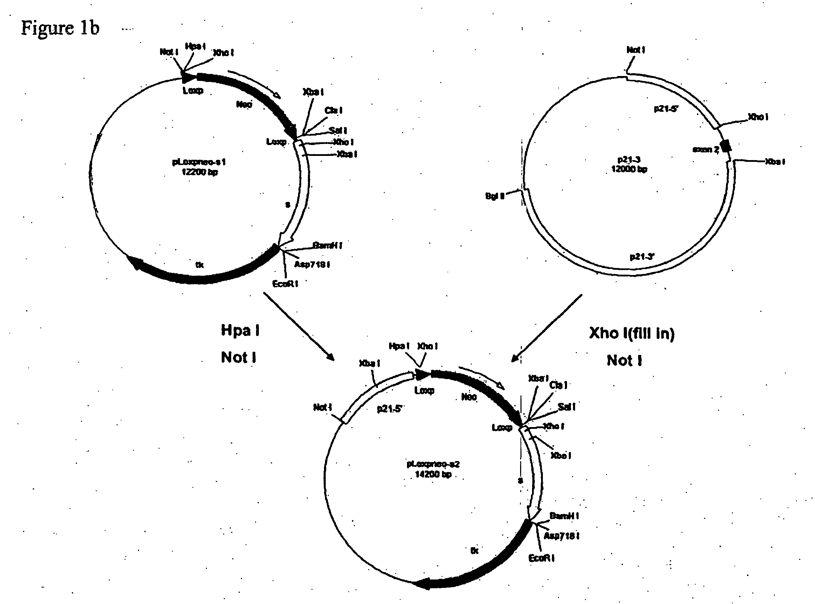

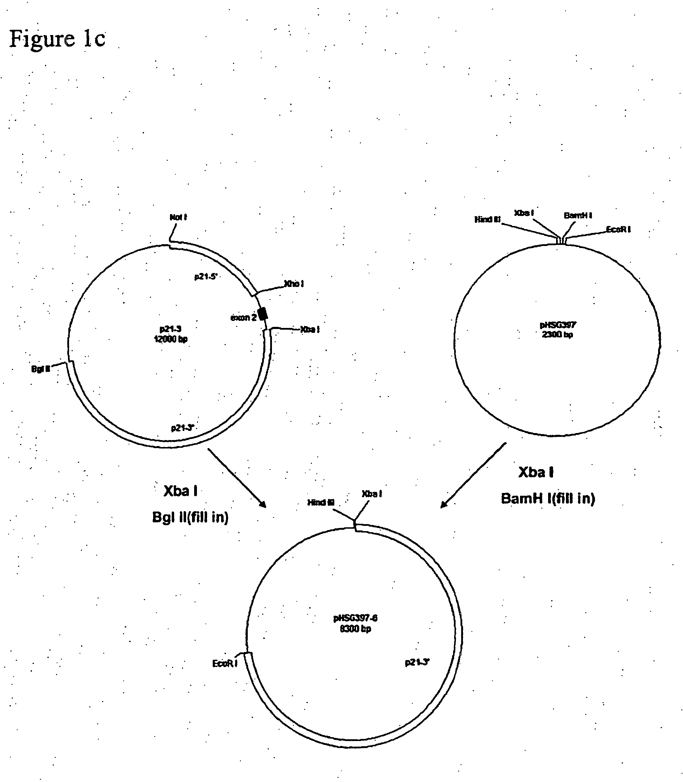

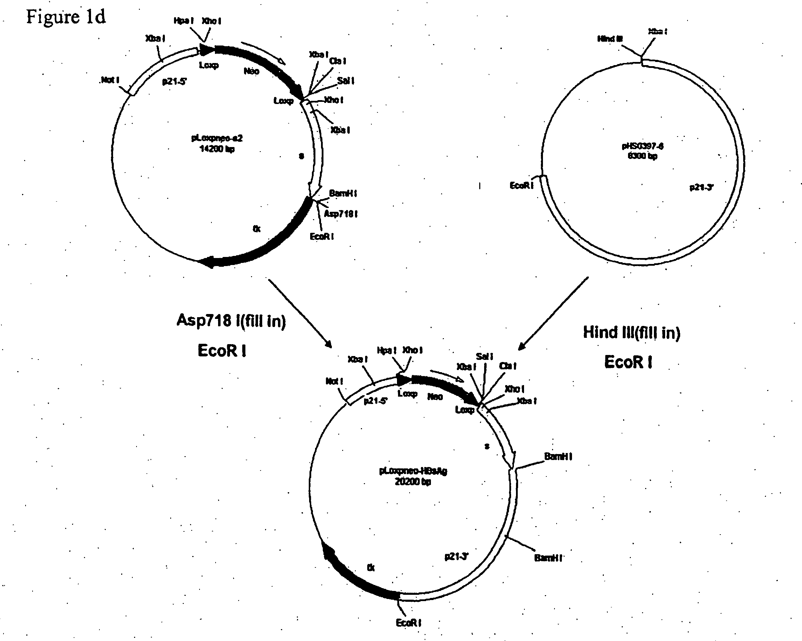

Construction of targeting vector containing a targeted integration of *HBsAg* gene

pADR-1[8] comprising HBV cDNA was digested with BglII and BamHI. A 2.2kb fragment comprising full length *HBsAg* gene was isolated and inserted into the BamHI site of vector pLoxpneo to obtain vector pLoxpneo-s1. A 2.0kb Xho I(filling in)-Not I(from clone vector) fragment upstream the second exon of p21 locus as short arm of homologous sequences was inserted between Hpa I site and Not I site of pLoxpneo-s1 to obtain vector pLoxpneo-s2. A 6.0kb Xba I -BglII fragment downstream the second exon of p21 locus was cloned to vector pHSG397 to generate HindIII(filling in) and EcoRI sites. Finally, the fragment as long arm of homologous sequences was inserted between Asp718 I(filling in) and EcoRI sites of vector pLoxpneo-s2 (figure 1), to generate targeting vector pLoxpneo-HBsAg containing a targeted integration of *HBsAg* gene.

Example 2

Construction of targeting vector containing a targeted integration of HBV X gene

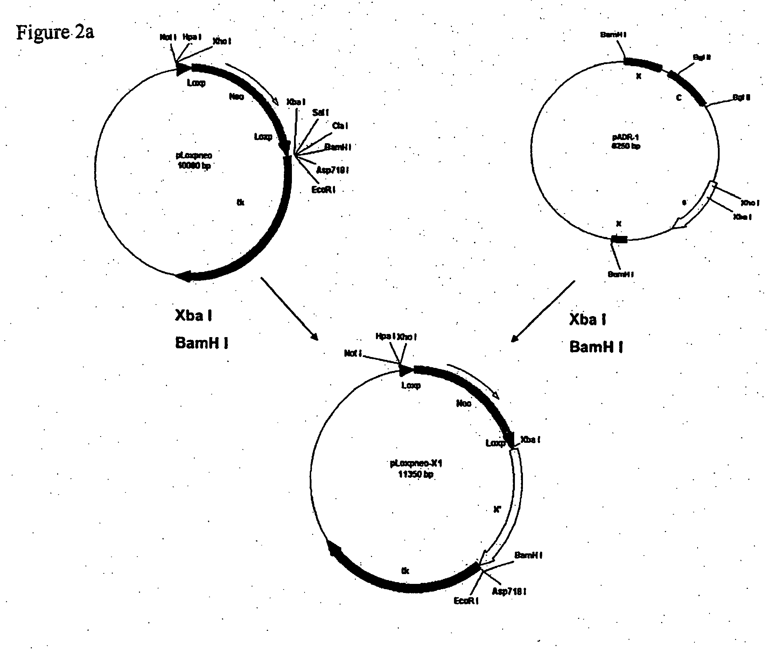

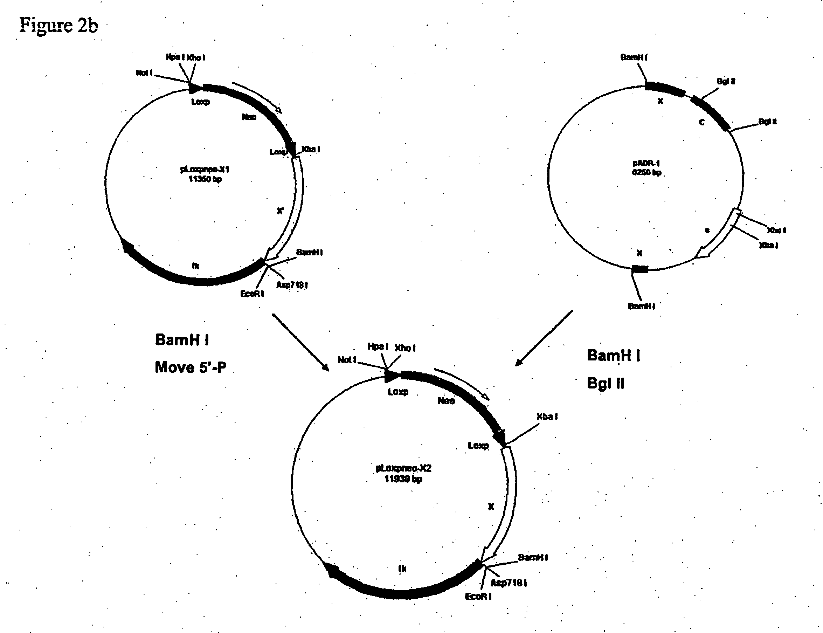

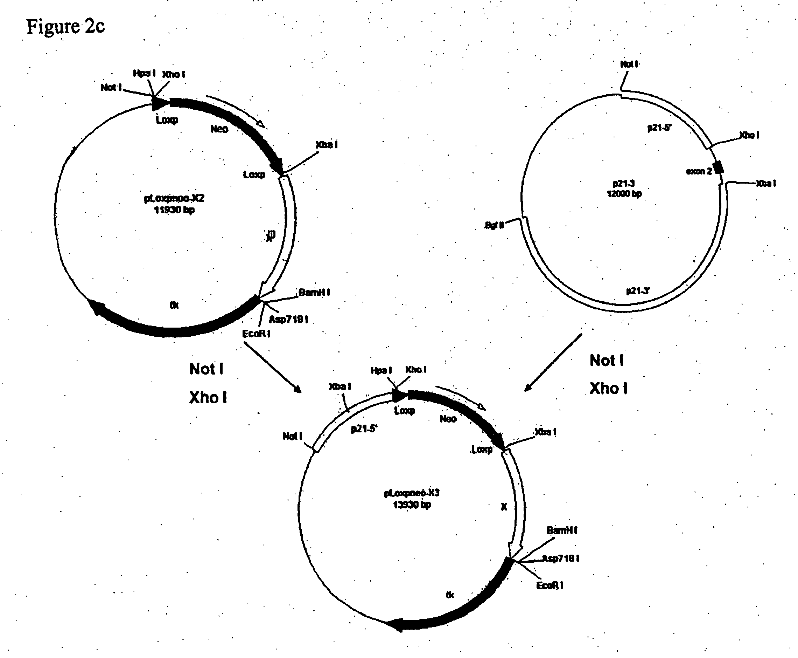

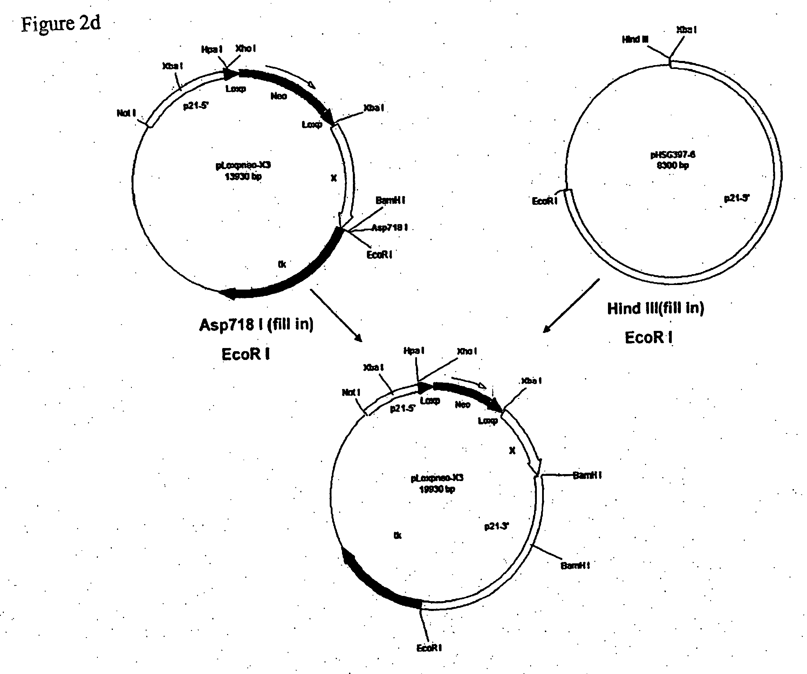

pADR-1 comprising HBV cDNA was digested with XbaI and BamHI. A 1.15kb fragment comprising HBX gene 5' end and a portion flanking X gene 5' was isolated and inserted into vector pLoxpneo to obtain vector pLoxpneo-X1. pADR-1 comprising HBV cDNA was digested with BglII and BamHI. A 0.58kb fragment comprising 3' end of HBX gene was isolated and inserted into vector pLoxpneo-X1 to obtain vector pLoxpneo-X2 containing entire HBX gene and a portion flanking its 5' end. A 2.0kb Xho I-Not I fragment upstream the second exon of p21 locus as short arm of homologous sequences was inserted between Xho I site and Not I site of the vector to obtain vector pLóxpneo-X3. A 6.0kb Xba I -BglII fragment downstream the second exon of p21 gene was cloned to vector pHSG397 to generate HindIII (filling in) and EcoRI sites. Finally, the fragment as long arm of homologous sequences was inserted between Asp718 I(filling in) site and EcoRI site of vector pLoxpneo-X3 (Figure 2), to generate targeting vector pLoxpneo-HBX containing a targeted integration of *HBX* gene.

Example 3

Generation of mouse embryo stem cells containing a targeted integration of HBsAg gene

*1) Preparation of primary mouse embryonic fibroblasts to serve as feeder cells*

1. 14 days postpregnancy mice were sacrificed, opened abdominal cavities, taken out uteri with embryos. Embryos were dissected from uteri in PBS, then placed in sterile 100 mm Petri dishes.

2. Yolk sac, amnion, and placent were removed, and embryos were subject to washing twice in fresh PBS.

3. The heads and visceras of embryos were removed by pairs of forceps, and fetal mice were cut into small pieces (1-3 mm$^3$) by sterile scissor, then washed with PBS until PBS was substantially colorless.

4. The tissue pieces were transferred to 15 ml centrifuge tubes containing PBS, centrifuged at 1000 rpm for 2 minutes.

5. Supernatant was discarded, and 5 ml of 0.25% trypsin was added to the remains, treated at 37°C for 30 minutes, then added DMEM medium supplemented with 10% fetal calf serum, and pipetted up and down repeatedly.

6. Tubes were centrifuged at 1000 rpm for 2 minutes.

7. Supernatant was discarded. After adding feeder cell medium, cells were transferred to 150 mm Petri dishes, cultured in an incubator with an atmosphere of 5%$CO_2$, at 37°C for 2-3 days. Usually embryonic fibroblasts would cover Petri dishes in 3 days.

8. Embryonic fibroblasts in one Petri dish were distributed into three Petri dishes, three days later frozen using feeder cell medium plus 10% DMSO. The cells were primary mouse embryonic fibroblasts.

*2) Treatment of primary mouse embryonic fibroblasts*

Primary mouse embryonic fibroblasts should be treated with mitomycin C before they were used as feeder cells, in order to arrest their growth, so that they could support the growth of ES cells without overgrowing cultured ES cells.

1. A tube of primary mouse embryonic fibroblasts was quickly thawed at 40°C water bath, then cells were transferred to a centrifuge tube containing 2 ml medium, centrifuged at 1000 rpm for 2 minutes.

2. Supernatant was discarded. After being added fresh feeder cell medium, cells were transferred to two 100mm Petri dishes, incubated in an incubator with an atmosphere of 5%$CO_2$ at 37°C.

3. Three days later, cells were treated with trypsin, then transferred to six 150 mm Petri dishes, incubated at 37°C for 3 days.

4. Cells were transferred from six 150 mm Petri dishes to twelve 150 mm Petri dishes, incubated at 37°C for 3-4 days.

5. Cells were transferred from twelve 150 mm Petri dishes to forty 150 mm Petri dishes, incubated at 37°C for 3-4 days.

6. 3.3ml PBS was added to a vial of mitomycin C(2 mg), with 3 vials of mitomycin C collecting a total of 10ml. To each Petri dish was added 0.25 ml mitomycin C to final concentration of 10 $\mu$g/ml, then incubated at 37°C for 2-3 hours.

7. To each Petri dish, medium was discarded, then cells were washed once with PBS, treated with 3ml of 0.25% trypsin. 10 minutes later, 5 ml of feeder cell medium was added to end reaction.

8. Cells from all Petri dishes were collected into 50 ml centrifuge tubes. These tubes were centrifuged at 1000 rpm for 2 minutes.

9. 40 ml of freezing medium (15% FCS, 10% DMSO, 75% DMEM) was added to the cells. After being pipetted up and down repeatedly, cells were rapidly divided into 40 small-sized tubes, preserved at -80°C. Feeder cells so prepared usually can cover 3-4 of 90mm Petri dishes for each thawed small-sized tube.

Preparation of 600 ml feeder cell medium can refer the following formula:

500ml DMEM
6ml L-glulamine
6ml non-essential amino acid solution (10mM)
6ml penicillin and streptomycin solution (5,000 unit penicillin, 5mg streptomycin /100ml)
4 l mercaptoethanol
90ml fetal calf serum
then the feeder cell medium was filtered and stored at 4°C. After adding 1000u/ml LIF to the medium, the medium can be used to culture ES cells.

*3) Transfection of ES cells by electroporation using targeting vector containing a targeted integration of HBsAg gene*

1. Transfection was performed at the second day (36 hr) after resuscitative ES cells were passaged.

2. The medium was changed into fresh medium 2 hrs before transfection.

3. The medium was discarded, then Petri dishes were washed twice with PBS.

4. 1.5 ml of 0.25% trypsin was added to each 100 mm Petri dish, 37°C for 5 minutes.

5. 3.5 ml of ES cell medium was added, pipetted up and down 20 times. Cell numbers were counted using blood cell counting chamber. For each targeting vector, it usually need about $2\times10^7$ cells/transfection.

6. ES cell suspension was centrifuged at 1000 rpm for 2 minutes, and supernatant was discarded. Cells were resuspended in 10 ml PBS.

7. Cell suspension was recentrifuged at 1000 rpm for 2 minutes, then cells were resuspended in PBS to make cell density up to $2\times10^7$ cells/ml.

8. 45 $\mu$g of linearized targeting vector from example 1 was mixed with 1 ml of cell suspension, then placed into electroporation cuvettes. The cuvettes were put at room temperature for 5 minutes.

9. Gene Pulsor(600 V, 25 $\mu$F) was discharged. The cuvette was removed and placed at room temperature for I minute.

10. Cells in electroporation cuvette were mixed with 7 ml fresh ES cell medium, then distributed into 4 Petri dishes (100 mm) covered with feeder cells.

11. 40 l of untransfected cells was placed in a 100 mm Petri dish as control.

12. 24 hours later, the medium was changed into selective medium containing G418 (280 $\mu$g/ml) and gancyclovir (2 $\mu$M) for screening ES cells, from then on, changed into fresh selective medium daily.

13. Individual clone was usually picked from day 7 after transfecting.

*4) Picking positive ES clones*

1. After being cultured in selective medium for 7-8 days and discarding the medium, the transfected ES cells were washed once with PBS, then changed into 10 ml PBS.

2. A 200 $\mu$l micropipettor was adjusted to 20 $\mu$l, then positive ES clones were picked using its tip under microscope.

3. Individual clones were transferred to an empty 96-well plate. For each 100 mm Petri dish, one usually can pick out 30-50 clones.

4. 50 $\mu$l of 0.25% trypsin-EDTA solution was added to each well, and reacted at 37°C for 3-5 minutes.

5. In each well, 100 $\mu$l of ES cell medium was added, pipetted up and down repeatedly with micropipettor tips to make ES clones into single cell suspension, wherein the micropipettor was a 100 $\mu$l eight-channel one and had adjust to 80 l. Single cell suspension from each ES clone was divided into two aliquots and replated onto two 96-well plates with feeder cells respectively. For the two plates, each clone' position and order were the same. Following alteration of the medium into fresh ES cell medium daily and culturing for 2 to 3 days, one plate was frozen, the other one was trypsinized and ES clones in it were transferred to 24-well plates, cultured with feeder cells medium until the medium color turned into yellow.

*5) Freezing positive ES clones*

1. The medium was discarded, then 100 $\mu$l of PBS was added.

2. After PBS being discarded, 50 $\mu$l of 0.125% trypsin-EDTA solution diluted with PBS was added, and reacted at 37°C for 3-5 minutes.

3. 100 $\mu$l of freezing medium (15% DMSO, 20% fetal calf serum, 65% DMEM) was added.

4. Cells were pipetted up and down for at least 10 times with an eight-channel micropipettor. The 96-well plate was sealed with sealing membrane and closed in a plastic bag, with marks being made on the surface of the plate and of the plastic bag.

5. To slow down freezing rate, the plate was first placed into a foam box, then froze at-80°C.

*6) Preparation ofgenomic DNA from ES clone*

1. ES cells were cultured in 12-well plates or 24-well plates. For ES cells used to extract genomic DNA, there is no need to add LIF to medium.

2. 400 $\mu$l of cell lysis buffer (0.5% SDS, 0.1M NaCl, 0.01M EDTA, 0.02M Tris-Cl pH7.6, protease K, 100$\mu$g/ml) was added to each well, then the plates were placed at room temperature for 5 minutes. The content in each well was transferred to an Eppendorf tube.

3. Tubes were kept at 50°C for 2 hrs.

4. 200 $\mu$l of saturated NaCl (6M) was added.

5. Eppendorf tubes were placed in cardboard box, and vigorously shaken 200 times.

6. Eppendorf tubes were placed on ice for 10 minutes.

7. The tubes were centrifuged at 14,000 rpm for 10 minutes under room temperature.

8. To each tube, 500μl of supernatant was transfer to a clean Eppendorf tube followed by adding 0.8 ml ethanol and mixing.

9. The tubes were centrifuged at 14,000 rpm for 5 minutes, and supernatant was discarded.

10. Each tube was put upside down and dried at room temperature. DNA was resuspended in 50-100μl of TE, left at 37°C till it had completely dissolved (24-48 hrs). The DNA can be used for PCR or Southern hybridization analysis.

*7) Identification of mouse embryo stem cells containing a targeted integration of HBsAg gene by Southern blot.*

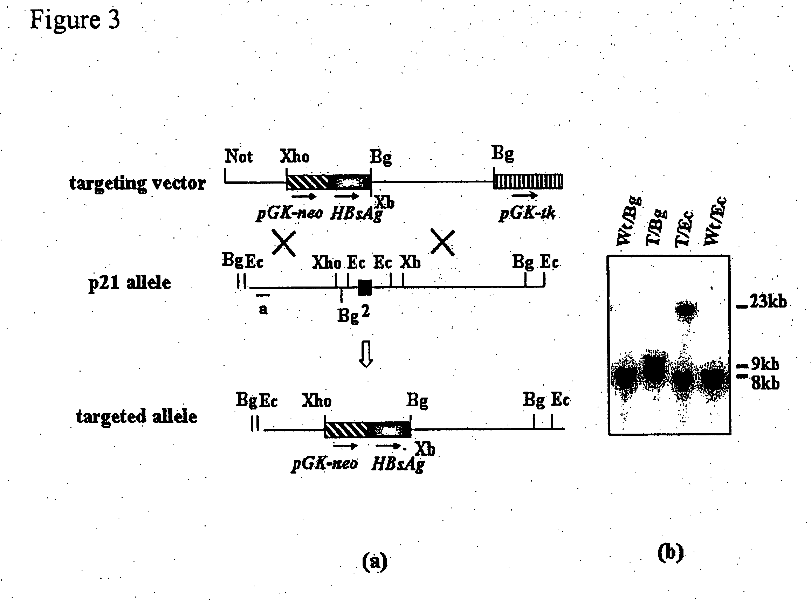

After extracted from G418/FIAU double resistant clone, genomic DNA was digested with EcoRI and Bgl II respectively, and the resultant digests were subject to Southern blot using probe a positioned outside 5' end of targeting vector. As regards digestion with Bgl II, a positive band approximately 8 kb should appear for wild type p21 allele, whereas this positive band would shift to approximately 9 kb for targeted p21 allele, the latter resulted from the deletion of an original Bgl II site and the introduction of another Bgl II site via neo gene after homologous recombination. As regards digestion with EcoRI, a positive band approximately 7.7 kb should appear for wild type p21 allele, whereas this positive band containing *HBsAg* gene would shift to approximately 22 kb(figure 3) for targeted p21 allele.

Example 4

Preparation of mouse embryo stem cells containing a targeted integration of HBV X gene

By a method similar to that as described in Example 3, mouse embryo stem cells containing a targeted integration of HBV X gene can be obtained.

Example 5

Preparation of transgenic mouse containing a targeted integration of *HBsAg* gene

*1) Preparation of donor blastulas*

The number of blastulas depends on many factors, such as mouse lines, general health state, adaption, capability of superovulate and experiment season et al. To obtain more blastulas or when the number of mice is limited, the unexcited mice are typically intraperitoneally injected with hormone to make them ovulate more ovas, i.e. superovulate. Mouse to be superovulated is usually at 3-5 weeks of age, being in adolescence, and the specific age may vary according to mouse lines, for exsample, the most suitable age for female C57BL/6J mice is at the age of 25 days.

*Preparation of blastulas from superovulated mouse :*

1. Unexcited 4-5 week old female C57BL/6J mice (12.5-14g) were selected, intraperitoneally injected 5IU of pregnant mare serum gonadotropin (PMSG, SIGMA) at 2:30 pm on day 1.

2. Before noon on day 3, i.e. within 48 hours of the last PMSG injection, female mice were intraperitoneally injected 5IU of human chorionic gonadotropin (HCG, SIGMA) to superovulate, then transferred to cages with stud male mice and mated with them.

3. Before 9:00 am on day 4, the presence of copulatory plug was checked. Mice that had copulatory plug were used as donor female mice and placed in other cages, that day was designated as day 0.5. At 2:30 pm on the same day, excited white female Kunming mice (about 24-30g) were selected to be mated with spermaductus-deligated male mice.

4. Before 9:00 am on day 5, the presence of copulatory plug was checked. Mice that had copulatory plug were used as acceptor female mice and placed in other cages.

5. On day 7, i.e. on day 4 after mating, blastulas were collected from uteri of donor female mice.

*Collection and culture of blastulas:*

1. After donor female mice were sacrificed by luxation, their abdomens were sterilized with 70% alcohol. A horizontal incision was made at the middle line of abdomen.

2. Skin was cut open and pulled to the lateral sides of the incision, then peritoneum was raised and cut open to expose abdominal cavity sufficiently.

3. Bowel tissues were raised above peritoneum to find uterus, then uterus was excised carefully at the place where both oviducts connect with uterine extremities, and placed at a sterile 60mm Petri dish.

4. Oviducts and uterine extremities were removed from uterine head and uterine tail respectively, to make separate uterus unobstructed.

5. Enough Brinster's BMOC-3(GIBCO BRL) medium was taken in a 5ml of single-use injection syringe. After a 5 gauge needle was put on, the injection syringe was inserted into uterine cavity under the dissecting microscope, then syringe plug was pushed to flush uterine cavity in different directions. uterus might expend slightly during flushing, and mouse embryos quickly sank to the dish bottom.

6. Several drops of medium was dripped in a 35 mm Petri dish, with each droplet about 200 μl, then covered with mineral oil (SIGMA, M-3516).

7. Embryos were collected using a flushing pipette under the dissecting microscope and transferred to culture microdrops, then incubated in an incubator with an atmosphere of 5%$CO_2$ for 2 hours at 37°C.

*2) Microinjection of blastulas*

Microinjection of ES cells should use phase contrast microscope or differential interference microscop(Nikon) with the effect of depth and solidity. Micromanipulators (Narishige), equipped with at least a low magnifying objective and a high magnifying objective to magnify at 200x, and further with a 37°C homoiothermy object stage, had better over an anti-seismic platform.

*Preparation of pipettes for microinjection:*

Before ES cells is injected into blastulas, various kinds of pipettes need prepared for blastula manipulation, including transfer pipette for transferring embryo, holding pipette for fixing embryo during microinjection, and injection pipette for injecting cells into blastula.

*Preparation of holding pipette for microinjection using needle-making burner (Narishige, MF-900):*

1. A thin glass capillary (Nikon, G-1) was pulled over small flame into a thin needle about 2-3 cm in length.

2. Glass capillary was melt on the filament of needle-making burner to become a glass ball. The prepared needle was fixed and adjusted to horizontal with filament. Filament was heated, then glass needle was allowed at its 60-90μm diameter to approach and contact glass ball

3. After filament's heating stopped, needle broke, as it contracts when it becomes cool, to form a blunt holding pipette.

4. Again filament was heated, then needle tip was allowed to approach it in order to make needle tip blunt, with 12-20mm mid-aperture.

5. Needle was heated at 1cm away from needle tip to make it bent at an angle of 30°, thus needle tip may be horizontal in micromanipulation field.

*Preparation of injection pipette using needle-making burner:*

1. Glass capillary was pulled into a thin needle about 1cm in length.

2. The needle was allow to break at 12-15μm of inner diameter using needle-making burner, which inner diameter just can hold one ES cell, with the ES cell not being crushed.

3. The needle was allow to bent at a little away from needle tip to obtain an angle of 30°, thus needle tip may be horizontal in micromanipulation field.

Preparation of transfer pipette was similar to that of holding pipette, the only difference lying in that the inner diameter of transfer pipette is 1-1.5 times as much as that of blastula (about 110-130μm), thus blastula can in and out of it readily. The needle length is about 2-3cm to hold medium, bubble, and a certain number of blastulas.

*Manipulation procedures of blastula injection :*

1. After being filled with paraffin oil, the dish, the needle tube of holding pipette and that of injection pipette were adjusted to the same focusing horizontal level through fine tuning of a microscope.

2. ES cells to be injected were thawed several days before injection. The medium was changed into fresh ES medium on the morning of injection day. 1-2 hours later, the cells were trypsinized to prepare single cell suspension, then preserved in Brinster's BMOC-3 medium.

3. About 10 blastulas with plump morpha, clear border, bigger blastocoele were selected from 35 mm Petri dish, transferred to injection chamber mounted holding pipette and injection pipette, then a few ES cells were sucked using flushing pipette into the chamber.

4. 12-15 small and round ES cells were sucked using injection pipette under a 10x magnification.

5. Holding pipette was allowed to attach one side of blastula under a 40x magnification, then injection pipette was adjusted to the same horizontal level as that of the center of blastula.

6. Operating stick of injection pipette was moved to make injection pipette quickly pierce through the wall of blastula into blastocoele, then injection pump was pushed to allow ES cells to enter blastocoele in sequence, finally pulled out carefully.

7. An injected blastula then was placed at the other side of micromanipulation field, and the injection of the next blastula was continued.

8. All injected blastulas was sucked under 10x magnification to bigger dish using flushing pipette, and cultured in droplets plus Brinster's BMOC-3 medium, finally marks were made.

The rest might be deduced by analogy, and the number of blastulas to be injected was determined on the basis of the blastulas' condition and the number of acceptor mice on the day. Holding pipette and injection pipette were changed only when blocked or stuck readily. Droplets in microinjection chamber were refreshed a few hours after injection or when pH of the medium had altered.

*3) Implantation of embryos into uterus*

1. Acceptor female mice were weighed, intraperitoneally injected anesthetic (Avertin) according to their weights, and shaved off hairs on the back.

2. A transfer pipette was connected with a mouth-controlling tube, then sucked in medium, bubble, medium, bubble, injected blastula, bubble, and a little of medium in sequence.

3. The back of each acceptor mouse was sterilized with 75% alcohol, then a 1cm horizontal incision was made at the right side near the first lumbar vertebra. Skin was pulled to the lateral sides of incision till ovary on the right and its fat pad could be seen through peritoneum. A 3mm opening on peritoneum was torn using a cuspidal forceps.

4. After fat pad being clamped out with left hand, uterus could be seen. Fat pad was fixed using a small-sized hemostatic forceps.

5. Uuterine wall was clamped at a place 2mm away from interface between uterus and oviduct with a cuspidal forceps in left hand, meanwhile a 4 gauge needle tip and a transfer pipette were hold in right hand. An opening was pierced at a place near to the cuspidal forceps and away from blood vessel, using needle tip under the dissecting microscope, then into it was inserted the front part of a transfer pipette carefully, and embryos were slowly blown into uterus.

6. Uterus and mesentery were sent back to abdominal cavity, then the incision was closed.

If the above operation was successful, pups would be born 17 days later, and it could be decued from their coat colors several days later that if highly chimeric mice had been obtained or not. When highly chimeric mice were selected to mate with C57BL/6J mice, transgenic offspring with pure brown coat would generate.

Example 6

Identification of transgenic mice containing a targeted integration of *HBsAg* gene

*1) Preparation of mouse genomic DNA*

1. About 0.5cm pieces of tail tip was cut from 15 day old mice, then placed in Eppendorf tubes.
2. To each tube, add 400 μl of lysis buffer (0.5% SDS, 0.1M NaCl, 0.05M EDTA, 0.01M Tris-Cl pH8.0, protease K, 200 μg/ml) to lyse tail tip.
3. The tubes were incubated overnight at 50°C.
4. 200 μl of saturated NaCl (6M) was added to each tube.
5. Eppendorf tubes were placed in cardboard box, and vigorously shaken 200 times.
6. Eppendorf tubes were placed on ice for 10 minutes.
7. The tubes were centrifuged at 14,000 rpm for 10 minutes under room temperature.
8. To each tube, 500 μl of supernatant was transferred to a clean Eppendorf tube followed by adding 0.8 ml ethanol and mixing.
9. The tubes were centrifuged at 14,000 rpm for 5 minutes, and supernatant was discarded.
10. Each tube was put upside down and dried at room temperature. DNA was resuspended in 50-100 μl of TE, left at 37°C till it had completely dissolved (24-48 hrs). The DNA can be used for PCR or Southern hybridization analysis.

*2) PCR identification of genotype in mice*

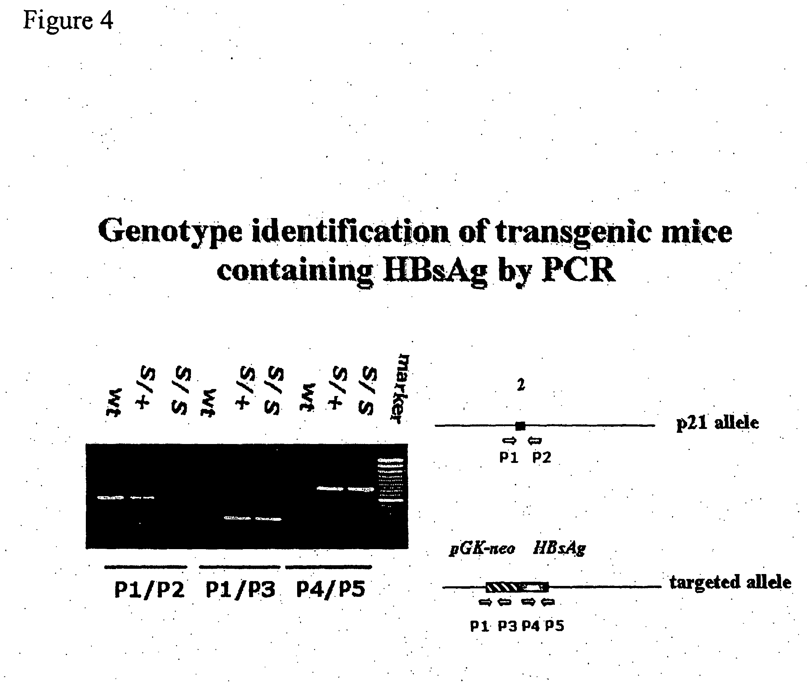

Primer 1(5'-TCTTCTGTTTCAGCCACAGGC-3') and primer 2(5'-TGTCAGGCTGGTCTG CCTCC-3') were used to identify wild type p21 allele, and a 436bp band could be amplified from wild type and heterozygous mice. Primer 3 (5'-ATTTTCCAGGGATCTGACTC-3') and primer 4 (5'-CCAGACTGC CTTGGGAAAAGC-3') on neo gene were used to identify targeted allele containing neo gene. Primer 5 (5'-GGAC CCTGCACCGAACATGG-3') and primer 6 (5'-GGAATAGCCCCAACGTT TGG-3') were used to identify *HBsAg* gene(figure 4).

*3) Identification of mice containing a targeted integration of HBsAg gene using Southern blot hybridization*

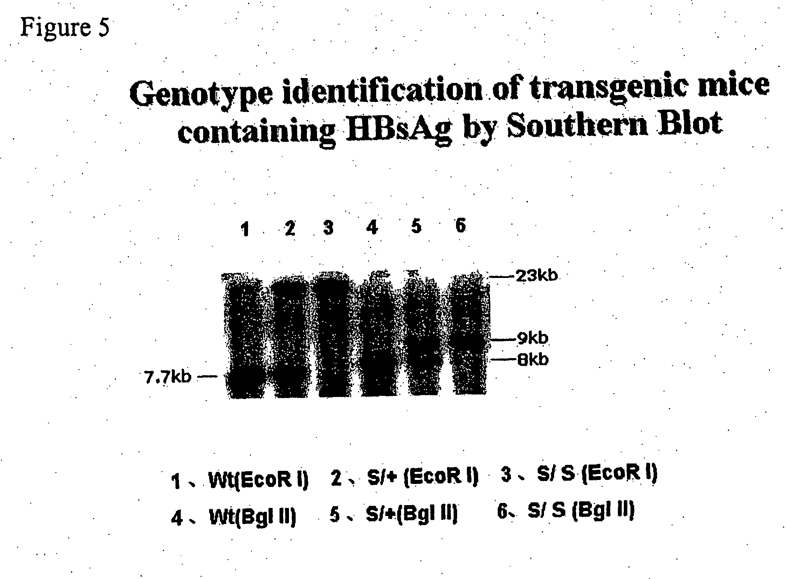

Mouse tail genomic DNA extracted was digested with EcoRI and Bgl II respectively, and the resultant digests were subject to Southern blot using probe a positioned outside 5' end of targeting vector. As regards digestion with Bgl II, a positive band approximately 8 kb should appear for wild type p21 allele, whereas this positive band would shift to approximately 9 kb for targeted p21 allele, the latter resulted from the deletion of an original Bgl II site and the introduction of another Bgl II site via neo gene after homologous recombination. As regards digestion with EcoRI, a positive band approximately 7.7 kb should appear for wild type p21 allele, whereas after mutation, the band containing *HBsAg* gene would shift to-approximately 22 kb for targeted p21 allele (figure 5).

*4) Extraction of total RNA from tissue*

1. After being added 2ml of Trizol, 100mg mouse tissue (liver, spleen, kidney et al.) was homogenized with a homogenizer, then left at room temperature for 5 minutes.
2. 0.4ml of chloroform was added The tube was closed tightly, shaken vigorously for 15 seconds, and left for 2-3 minutes, then centrifuged at 10,000-12,000g for 15 minutes at 2-8°C.
3. The aqueous upper layer was transferred to a fresh centrifuge tube, then added 1ml of isopropanol and mixed. The tube was placed at room temperature for 10 minutes followed by centrifugation at 10,000-12,000g for 10 minutes at 2-8°C.
4. Supernatant was discarded. Then the pellet was washed by adding at least 2ml of *75%ethanol*, centrifuged at a speed no more than 7500g for 5 minutes at 2-8°C.
5. Supernatant was discarded. The RNA pellet was air dried for 5-10 minutes, then dissolved in 500μl of RNase-free water by pipetting repeatedly.
6. The RNA was kept at 55-60°C for 10 minutes, then stored at -70°C.

*5) Identification of mice containing a targeted integration of HBsAg gene using Northern hybridization*

1. 1.5g of agarose was dissolved in 117.2ml DEPC-treated water, then cooled to 70°C, added 3.5 l of 10mg/ml ethidium bromide (EB), 30ml of 5×MOPS (0.1mol/l MOPS(pH7.0), 40mmol/l sodium acetate, Smmol/IEDTA (pH8.0)) and 2.8ml of 40% formaldehyde solution.

2. 20μg of RNA was added to 3× volumes of loading buffer(6.7ml of formamide, 2.2ml of 40% formaldehyde, 1320 l of 10×MOPS, 20 l of 20mg/ml bromophenol blue), heated to 60°C for 10 minutes, and chilled quickly in ice water, then loaded the sample.

3. Electrophoresis apparatus was attached to an electric power supply. A voltage of 120-160v was applied to the gel and ran for 2-3 hours until the bromophenol blue shifted 8cm away the loading well. During this period, bipolar electrophoresis buffers were mixed every half hour.

4. The gel was immersed in 20×SSC twice, each time for 15 minutes.

5. RNA was transferred from gel to nitrocellulose membrane using 20×SSC, then the membrane was washed with 2×SSC for 5 minutes.

6. After Crosslinked under UV (1200 W/cm$^2$) for 2 minutes, the membrane was baked at 80°C for 45 minutes.

7. A probe was labeled using Primer A Labeling System(Cat #U1100) from Promega Corp. Briefly, DNA fragment to be labeled was boiled at 95-100°C for 2 minutes, then rapidly chilled on ice water. The following solutions were mixed in a 1.5ml centrifuge tube:

Table 1

| solution | volume | final concentration |
|---|---|---|
| 5×buffer | 10μl | 1× |
| dNTP(without dCTP) | 2μl | each dNTP 20μM |
| DNA fragment to be labeled* | 25ng | 500ng/ml |
| [α-32p]dCTP,50μCi | 5μl | 333nM |
| DNA polymerase I (Klenow fragment) | 5 units(1μl) | 100 units/ml |
| * DNA fragment to be labeled is a 550bp fragment recovered from Sph I and Spe I double-digested plasmid pADR-1. | | |

Then nuclease-free water was added to a volume of 50 l and mixed. Standing at room temperature for 1 hour and boiling for 2 minutes, the tube was then quickly chilled on ice water, then added EDTA to final concentration 20mM to end reaction

8. The membrane was immersed in 6×SSC for 5 minutes, then incubated at 65°C for 2 hours in prehybridization solution (6×SSC, 2×Denhardt, 0.1%SDS, 100 g/ml of denatured salmon sperm DNA). After the labeled probe was added into prehybridization solution, hybridization continued at 65°C for more than 18 hours.

9. The membrane was washed in 1×SSC, 0.1% SDS at room temperature for 20 minutes, then in 0.2×SSC, 0.1%SDS at 65°C for three times, each time for 20 minutes.

10. The membrane was slightly dried, then exposed to X-ray film at -70°C.

11. The film was developed.

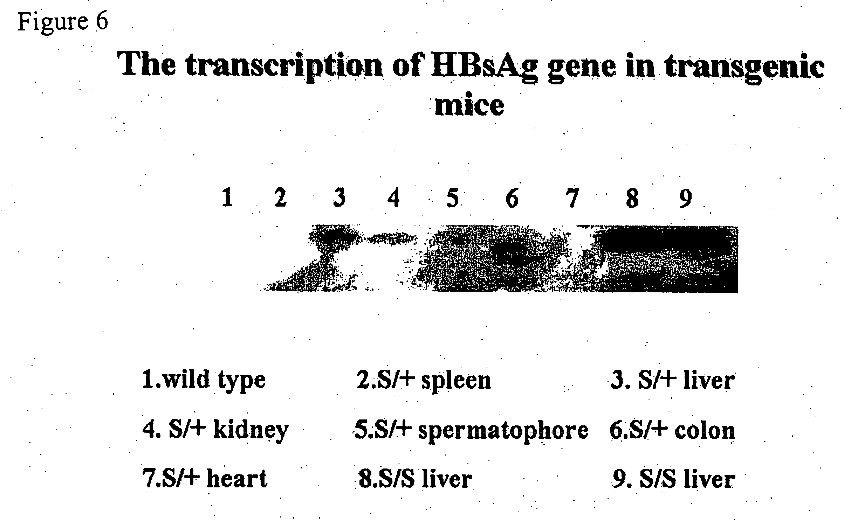

Results were shown in figure 6.

*6) Identification of mice containing a targeted integration of HBsAg gene using ELISA*

One-step ELISA kit for HBsAg was purchased from Sihuan Biological Corp., Beijing, and the following steps were used:

1. loading samples:

Microtiter plates were coated. To each plate, two wells were set as negative control and as positive control respectively, then a drop of control solution (50μl) was added to each of the four wells. One well was set as blank control (without any agents being added). 50μl of tested samples was added to the other wells. Then a drop (50μl) of anti-HBs-HRP was added to each well. The plates were incubated at 37°C for 30 minutes.

2. Washing plates :

Liquid in each well was discarded, then the wells were washed 5 times using 30× diluted washing buffer, and patted to make dry.

3, Developingcolor:

A drop of chromogenic solution A and a drop of chromogenic solution B were added to each well, then the plates were incubated at 37°C for 15 minutes to develop color.

4, Ending reaction:

A drop of termination buffer was added to end reaction.

5, Reading results :

Blank well was calibrated at a wavelength of 450nm, then OD value of each well was read, and results were calculated according to the following formula:

S/N=OD value of tested serum/average OD value of negative control, when S/N≥2.1, the tested serum was regarded as positive, when S/N<2.1, the tested serum was regarded as negative. If OD450 of negative control <0.05, then 0.05 was took in the formula; If OD450≥0.05, then actual value was took in the formula. Results were as follows:

Table 2

| Sample | Positive control 1 | Positive control 2 | Negative control 1 | Negative control 2 | Wt serum 1 | Wt liver homogenate 1 | Wt serum 2 | Wt liver homogenate 2 |
|---|---|---|---|---|---|---|---|---|
| OD value | 1.777 | 1.797 | 0.017 | 0.016 | 0.015 | 0.019 | 0.015 | 0.017 |
| S/N | 35.54 | 35.94 | 0.34 | 0.32 | 0.30 | 0.38 | 0.30 | 0.34 |

Table 3

| S+/- serum 1 | S+/- liver homogenate 1 | S+/- serum 2 | S+/- liver homogenate 2 | S+/+ serum 1 | S+/+ liver homogenate 1 | S+/+ serum 2 | S+/+ liver homogenate 2 |
|---|---|---|---|---|---|---|---|
| 0.015 | 1.908 | 0.016 | 1.879 | 0.013 | 2.067 | 0.015 | 1.998 |
| 0.30 | 38.14 | 0.32 | 37.58 | 0.26 | 41.34 | 0.30 | 39.94 |

Example 7

Identification of transgenic mice containing a targeted integration of HBV X gene

*1) Identification of genotype of mouse by PCR*

Primer 1(5'-TCTTCTGTTTCAGCCACAGGC-3') and primer 2 (5'-TGTCAGGCTGGTCTG CCTCC-3') were used to identify wild type p21 allele, and a 436 bp band could be amplified from wild type and heterozygous mice. Primer 3 (5'-ATTTTCCAGGGATCTGACTC-3') and primer 4 (5'-CCAGACTGC CTTGGGAAAAGC-3') on neo gene were used to identify targeted allele containing neo gene. Primer 7 (5'-TCTCTGCCAAGTGTTTGCTGACGC-3') and primer 8 (5'-TCGGTCGTT GACATTGCTGAGAGTC-3') were used to identify *HBX gene.*

*2) Identification of mice containing a targeted integration of HBX gene by Southern blot*

Extracted mouse tail genomic DNA was digested with EcoRI and Bgl II respectively, and the resultant digests were subject to Southern hybridization using probe a positioned outside 5' end of targeting vector. As regards digestion with Bgl II, a positive band approximately 8 kb should appear for wild type p21 allele, whereas this positive band would shift to approximately 9 kb for targeted p21 allele, the latter resulted from the deletion of an original Bgl II site and the introduction of another Bgl II site via neo gene after homologous recombination. As regards digestion with EcoRI, a positive band approximately 7.7 kb should appear for wild type p21 allele, whereas after mutation, the band containing *HBX* gene would shift to approximately 22 kb for targeted p21 allele.

*3) Identification of mice containing a targeted integration of HBX gene by Northern blot*

A 580bp fragment, which was recovered from Bgl II and BamHI digestion products of pADR-1 containing HBV cDNA, was used as probe for Northern hybridization. Results showed that *HBX* gene was expressed in the liver and kidney of mouse.

<u>Example 8</u>

<u>Phenotype analysis of transgenic mice containing a targeted integration of *HBsAg* gene</u>

*1) Hematoxylin and eosin staining*

1. Fixation

Tissues were fixed in neutral formaldehyde solution (100ml of 40% formaldehyde, 900ml of water, 4g disodium phosphate, 6.5g sodium dihydrogen phosphate) over 20 hours.

2. desiccation

70% ethanol for 15 minutes
80% ethanol for 15 minutes
90% ethanol for 15 minutes
95% ethanol for 15 minutes
anhydrous alcohol for 15 minutes
anhydrous alcohol for 15 minutes
xylene for 15 minutes
xylene for 15 minutes

3. embedding

Desiccated tissues were transferred into treated paraffin (i.e. being melt and placed overnight at 60°C), and immersed for 2 hours, with paraffin changed twice during this period. Then tissues were transferred to preheated embedding framework followed by melting paraffin being poured in quickly, meanwhile tissue's position being adjusted using warm forceps. Floorplate then was put on, on it was poured some melting paraffin, and bubbles in it were carefully removed. They were placed at room temperature to solidify melting paraffin.

4. Section cutting

Lump paraffin was made to suitable size, successively chipped at a rotary microtome (MICROM HM340E), which blade had been adjusted to an angle of 10°. The formed wax-chips were moved to a clean aluminium foil, cut to suitable size using surgical blade and transferred to slides respectively. Proper amount of water was added to allow sample to float on the surface of water, then the slides were placed on the platform of slide-drying machine at about 40°C, and discarded water carefully until sample had outspread. The slides were collected in specimen boxes, dried overnight at 37°C.

5. Hematoxylin and eosin staining

xylene for 15 minutes;

xylene for 15 minutes;
anhydrous alcohol for 15 minutes;
95% ethanol for 5 minutes;
70% ethanol for 5 minutes;
water for 5 minutes;
Harris hematoxylin staining solution (2.5g of hematoxylin, 25 ml of anhydrous alcohol, 50g of potassium alum, 1.25g of mercuric oxide, 20ml of glacial acetic acid, 500ml of water) for 5 minutes;
washed with running water for 5-10 minutes;
70% ethanol-hydrochloric acid(200ml of 70% ethanol+10 drops of hydrochloric acid) for 15-30 seconds;
70% ethanol-ammonium hydroxide (200ml of 70% ethanol+ 10 drops of ammonium hydroxide) for 1 minute;
water for 5 minutes;

Then the degree of discoloration was observed under microscope. If cytoplasm was gray or transparent, the next steps were carried out. If cytoplasm was yet blue, the above steps were repeated.

70% ethanol for 3 minutes;
eosin Y staining solution (0.5g of eosin Y, 50 mg of brilliant red B dissolved in 5ml water, 50 ml of water, 2ml of glacial acetic acid, 390ml of 95% ethanol) for 1 minute;
95% ethanol for 5 minutes;
anhydrous alcohol for 5 minutes;
xylene for 5 minutes;
xylene for 5 minutes;

6. Sealing the slides with neutral gum

Results were showed in figure 7.

*2) Immunohistochemical analysis*

1. Paraffin section was prepared, dewaxed to water routinely.
2. The slide was incubated in 3% hydrogen peroxide at room temperature for 10 minutes, in order to inactivate endogenous peroxidase.
3. The slide was washed 2 minutes×3 with distilled water, then placed into a vessel containing 0.01M citrate buffer (pH6.0). The vessel was heated in a microwave oven to make the buffer temperature at 92-98°C for 10-15 minutes, then taken out, cooled at room temperature for 10-20 minutes to renature antigen. The slide was immersed in PBS for 5 minutes.
4. Then the slide was blocked with 5-10% normal goat serum (PBS diluted), incubated at room temperature for 10 minutes, afterwards discarded serum, added primary antibody that had diluted to a suitable proportion (1% BSA-PBS diluted), incubated at 37°C for 1-2 hours or at 4°C overnight.
5. The slide was washed with PBS for three times, each time for 5 minutes.
6. Then the slide was added biotin-labeled secondary antibody diluted to a suitable proportion(PBS diluted), incubated at 37°C for 30 minutes.
7. The slide was washed with PBS for three times, each time for 5 minutes.
8. Then the slide was added horseradish peroxidase-labeled streptavidin diluted to a suitable proportion (PBS diluted), incubated at 37°C for 30 minutes.
9. The slide was washed with PBS for three times, each time for 5 minutes.
10. Color was developed using chromogenic agent.
11. The slide was washed sufficiently with water, counter-stained and sealed.

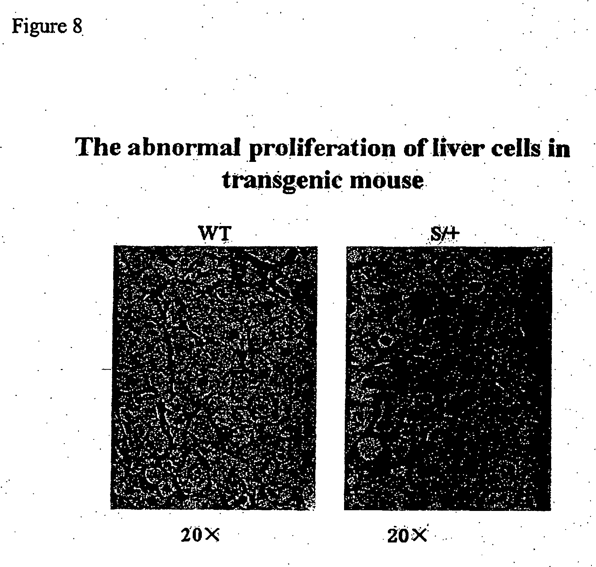

Results were showed in figure 8.

3) Assay of $\alpha$-fetal protein in mouse serum

The contents of $\alpha$-fetal protein in mouse serum were determined using quantitative ELISA kit for that of $\alpha\alpha$-fetal protein ($\alpha$-AFP) in human serum. The procedures were as follows:
1. Sera were separated from blood samples, stored at 4°C, avoiding repeatedly freezing and thawing.

2. Samples were added at the following ratio:

Table 4

| well type | standard well | sample well |
|---|---|---|
| standard ($\mu$l) | 20 | -- |
| Sample ($\mu$l) | -- | 20 |
| enzyme-conjugate ($\mu$l) | 100 | 100 |

mixed, then incubated at 37°C for 20 minutes. A well was set as blank in each experiment,

3. For each well, firstly the liquid in it was discarded, secondly distilled water was refilled in it, then discarded 10 seconds later. The step was repeated 3-4 times.

4. A drop of chromogenic agent A (50$\mu$l), a drop of chromogenic agent B (50$\mu$l) was add and mixed adequately, then placed at 37°C for 15 minutes for chromogenesis.

5. A drop of termination solution (50$\mu$l) was added. After being calibrated zero using blank well, the OD value of each well at a wavelength of 450nm was read.

6. A standard curve was plotted, using different concentrations' logarithm of standard as abscissa, corresponding OD value as ordinat. On the basis of the linear regression equation of the plotted standard curve, concentration of sample could be calculated via its tested OD value. The equation was as follow:

$$\text{logOD} = \text{Blog[concentration]} + A$$

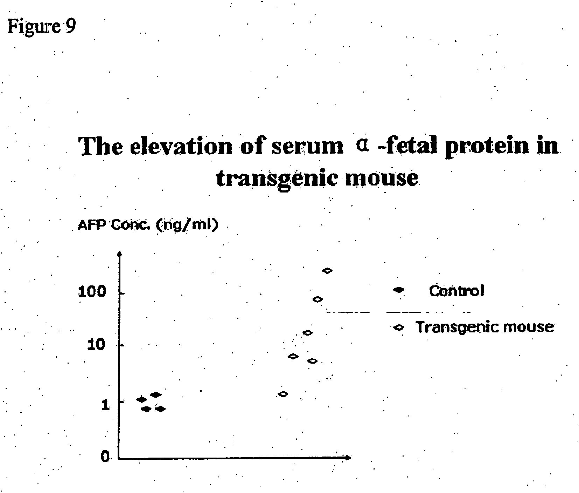

Comparative results of two month old mice were showed in figure 9.

*4) Detection of apoptosis*

ApopTaq (In Situ Apoptosis Detection Kit) from Oncor Corp. was used to detect apoptosis, procedures was as follows:

1. Paraffin section was dewaxed to water, immersed twice in PBS, each time for 5 minutes.

2. Pretreatment of tissue

The tissue was treated with PBS-diluted protease K(20$\mu$g/ml) at room temperature for 15 minutes (60$\mu$l/5cm$^2$), then washed with deionized water twice, each time for 2 minutes.

3. Inactivating endogenous peroxidase.

The tissue was treated with PBS-diluted 3% $H_2O_2$ at room temperature for 5 minutes, then washed with deionized water twice, each time for 5 minutes.

4. Equilibrating sample

The unwanted liquid was discarded carefully. 75$\mu$l of equilibration buffer was directly added onto sample, then incubated at room temperature for at least 10 seconds.

5. Incorporating TdTase

The unwanted liquid was discarded carefully. TdTase work solution(77$\mu$l of reaction buffer+33$\mu$l of TdTase) was directly added, then incubated at 37°C for 1 hour.

6. Ending reaction

Sample was added to termination buffer, stirred for 15 seconds, then continued incubation at room temperature for 10 minutes.

7. Adding antidigoxin-conjugate

Sample was washed three times with PBS, each time for 1 minute. After the unwanted liquid was discarded carefully, sample was covered with room temperature-warmed antidigoxin- peroxidase conjugate, then incubated at room temperature for 30 minutes.

8. Developing color

Sample was washed 4 times with PBS, each time for 2 minutes. DAB was used for chromogenesis.

9. Ending reaction using water

Sample was washed three times with deionized water, each time for 1 minute, then continued in deionized water for 5 minutes.

10. Counter-staining

0.5% methyl green (dissolving 0.5g methyl green in 100ml of 0.1M sodium acetate pH4.0) was used to counter-stain at room temperature for 10 minutes. Then sample was washed three times with deionized water, for the first twice, immersed in water for ten times each, for the third time, immersed in water for 30 seconds without stirring. Finally, sample was washed three times in 100% n-butanol, using above-mentioned method.

11. Sealing the slide

Sample was immersed three times in xylene, each time for 2 minutes, then sealed with neutral gum.

Results showed that obvious apoptosis wasn't detected in neither transgenic mice nor wild type mice at 15 months of age.

Example 9

Research on serum proteomics of mice containing a targeted integration of *HBsAg* gene

*1) Preparation of proteins from liver tissue*

1. Liver tissue was taken out as sample ;
2. The sample was powdered using morta under liquid nitrogen. After 0.5ml of lysis buffer (5M urea, 2M thiourea, 2% SB-30, 2% CHAPS, 1% DTT, a mixture of protease inhibitor) was added to per g sample, the sample was homogenized for 30 seconds with tissue homogenizer;
3. Tissue suspension was centrifuged at 10,000g for 10 minutes at 15°C ;
4. Its supernatant was supercentrifuged at 150,000g for 45 minutes at 4°C ;
5. Then the resultant supernatant was taken out, avoiding upper floating lipid layer carefully, again centrifuged at 40,000g for 50 minutes at 6°C ;
6. The resultant supernatant was taken out. After its protein concentration being determined according to Bradford method, the supernatant was stored in aliquots at -75°C.

*2) Preparation of mouse serum*

Bloods from 3 month old wild type mouse and transgenic homozygous mouse respectively, placed at room temperature for 30 minutes, then centrifuged at 3500g for 30 minutes. After upper serum being taken out and its protein concentration being determined using BCATM-Protein Assay Kit (Cat. No. 23226) from PIERCE Corp., each serum was stored in aliquots at -75 °C.

*3) Proteomics analysis of serum and liver tissue*

1. Isoelectric focusing

By using intragel rehydration method, serum or tissue extract containing 250 $\mu$g of protein was added to sample rehydration solution (8mol/l urea, 2%CHAPS, trace bromophenol blue) up to 350 $\mu$l of final volume, to which reductant DTT was added to 20mmol/l of final concentration, IPG buffer to 0.5% final concentration. An IPG dry gel(pH3-10L, 18cm, Amersham Pharmacia Biotech) was placed into above solution to carry out isoelectric focusing under the following parameters :

Table 5

| Step | Voltage (V) | Time (h) | Volt-hours(Vh) |
| --- | --- | --- | --- |
| rehydration | 0 | 12 | 0 |
| 1 | 500 | 1 | 500 |
| 2 | 1000 | 1 | 1000 |
| 3 | 8000 | 4 | 32000 |

2. Equilibration

After isoelectric focusing, the IPG gel was placed into 10ml of SDS equilibration buffer(50mmol/l Tris-Cl pH8.8, 6mol/l urea, 30% glycerol (V/V), 2% SDS (V/V), trace bromophenol blue), then shaked twice on a shaker, each time for 10 minutes, for the first time, the equilibration buffer contained 100mg DTT, for the second time, the equilibration buffer contained 254mg iodoacetamide.

3. The second dimension SDS-PAGE vertical electrophoresis

According to the size of IPG gel, a piece of 12.5% homogeneous gel with a size of $200 \times 200 \times 1mm^3$ was prepared. Equilibrated IPG gel was transferred onto SDS-PAGE followed by blockage with 0.5% agarose containing trace bromophenol blue, which was prepared by electrophoresis buffer (25mmol/l Tris, 250mmol/l glycine pH8:3, 0.1% SDS). At 14-15°C, First 20mA of constant current per gel was run for 40 minutes, then increased to 30mA until bromophenol blue shifted to 1mm away from inferior border of glass plate. The electrophoresis gel was fixed in methanol: acetic acid: water (3:3:4), stained with Coomassie brilliant blue staining solution (0.25% Coomassie brilliant blue R250, prepared by methanol:water:acetic acid=4.5:4.5:1,) over 4 hours. Then discolored using 7% acetic acid-10% ethanol.

4. Analysis of 2-D gel electrophoresis results

Electrophoresis result was analyzed by using image analysis software ImageMaster 2D Elite 3.01 from Amersham Pharmacia Biotech Corp.

5. Enzyme digestion and extraction of differential proteins from PAGE

Protein dots with obvious difference from 2D gel were selected, then cut off along staining border by blade, placed in tubes, discolored with a solution containing 50% acetornitrile (chromatograph grade), 50mM ammonium bicarbonate, centrifuged and dried under vacuum. 8-10$\mu$l of 0.01g/L trypsin solution was added to each tube, incubated at 37°C for 20 hours, in which trypsin was from Boehringer Corp. (modified, sequencing grade) and its solution was prepared by using 25mmol/L ammonium bicarbonate solution. 120$\mu$l of 5% trifluoroacetic acid (TFA, Fluka Corp.)was added, and incubated at 40°C for 1 hour. The supernatant was taken out, then 120$\mu$l of 2.5%TFA, 50% ACN solution were added, and incubated at 30°C for 1 hour, finally, combined and lyophilized.

6. Peptide mass fingerprinting(PMF) analysis of differential proteins

Peptide mixture was dissolved in 10 $\mu$l of 0.5% trifluoroacetic acid following lyophilization. 1 $\mu$l of the dissolved peptide mixture or desalted extract was taken to dot, and 1 $\mu$l of saturated 1-cyano-4-hydroxy cinnamic acid solution, dissolved in 0.1% trifluoroacetic acid/50% acetonitrile, was as matrix, then dried at room temperature. By using REFLEX III matrix assisted laser desorption/ionization time of flight mass spectrometry (MALDI-TOF-MS) from Bruker Corp., proteins were detected at 337nm of laser length.

7. Preliminary identification of protein through searching database

According to input data of protein isoelectric point, the range of molecular weight and peptide fingerprint profile and some other parameters, proteins matching there parameters could be searched among protein database taking the advantage of database searching program. Protein database searching programs on the web are as follows : PeptIdent provided by ExPASy Molecular Biology Server (http://www.expasy.ch/tools/peptident.html) and MS-Fit provided by UCSF Mass Spectrometry Facility (http://prospector.ucsf.edu/htmlucsf3.0msfit.htm).

**References:**

1. Bosch FX,Munoz N. Epidemiology of hepatocellular carcinoma. In: Bannsch P,Keppler D,eds. Liver cell carcinoma. Dordrecht: Kluwer Academic,1989;3-12.
2. Zuckerman AJ. More than third of word's population has been infected with hepatitis B virus[Letter]. Br.Med.J 1999 318:1213.
3. Gust ID. Epidemiology of hepatitis B infection in the Western Pacific and South East Asia. Gut 1996 38(suppl 2): s18-s23.

4. Chisari FV, Pinkert CA, et al. A transgenic mouse model of the chronic hepatitis B surface antigen carrier state. Science 1985 230:1157-1160.

5. Babinet C, Farza H, et al. Specific expression of hepatitis B surface antigen(HBsAg) in transgenic mice. Science 1985 230:1160-11.

6. Deng CX, Zhang P, et al. Mice lacking p21$^{CIP/WAFI}$ undergo normal development, but are defective in G 1 checkpoint control. Cell 1995 82:675-694.

7. Yang X, Li C, et al. The tumor suppressor SMAD4/DPC4 is essential for epiblast proliferation and mesoderm induction in mice. Proc. Natl. Acad. Sci. USA 1998 95:3667-3672.

8. Gan RB, et al. The complete nucleotide sequence of the cloned DNA of hepatitis B virus subtype adr in pADR-1. Sci. Sin. 30, 507-521 (1987).

**Claims**

1. A method for establishing a non-human embryo comprising:

   a) inserting an exogenous gene of interest into a suitable vector carrying sequences homologous to a target site, to construct a recombinant targeting vector;
   b) transfecting ES cells of a studied non-human animal using said targeting vector from step a), and screening targeted ES cells which have integrated the exogenous gene into a specific site;
   c) injecting targeted ES cells obtained from step b) into said non-human animal's blastulas and culturing the blastulas in vitro, so that embryos containing targeted ES cells are generated,

   wherein said exogenous gene of interest is a HBV or a HCV gene and said exogenous gene of interest is inserted into p21 locus of said non-human animal.

2. The method according to claim 1, wherein said exogenous gene of interest is a HBV gene

3. The method according to claim 2, wherein said exogenous gene of interest is HBsAg gene or HBV X gene.

4. The method according to claim 1, wherein said targeting vector comprises an exogenous gene of interest, homologous sequences of a target site, positive and negative selection markers.

5. The method according to claim 1, wherein said non-human animal is a mouse.

6. The method according to claim 5, wherein said exogenous gene of interest is inserted into the second exon of mouse p21 locus.

7. An isolated mouse cell, **characterized in that** an exogenous gene of interest is inserted into the p21 locus on the cell genome and wherein said exogenous gene of interest is a HBVor a HCV gene.

8. The cell according to claim 7 wherein the exogenous gene of interest is a HBV gene.

9. The cell according to claims 7-8, which is a embryo stem cell or somatic cell with the same genotype.

10. The cell according to claims 8 or 9, where said exogenous gene of interest is HBsAg gene or HBV X gene.

11. A transgenic non-human embryo obtained by the method of claim 1.

12. A transgenic mouse, which is **characterized by** the chromosome of said mouse being site-specifically integrated an exogenous gene of interest by gene knock-in method, wherein said exogenous gene of interest is HBV gene, and said exogenous gene of interest being inserted site-specifically into the p21 locus of said mouse.

13. A transgenic mouse according to claim 12, wherein said exogenous gene of interest is HBsAg gene or HBV X gene.

14. A transgenic mouse, which is **characterized by** HBsAg gene being inserted site-specifically into the p21 locus of said mouse, said exogenous gene of interest being stably inherited and expressed, said mouse progressing normally, and developing into hepatocellular carcinoma after certain time.

**15.** Use of the transgenic mouse according to any one of claims 12-14 in researching on hepatocellular carcinoma pathogenesis, its early stage diagnosis and therapy.

**16.** Use of the transgenic mouse according to any one of claims 12-14 for the screening of anticancer drugs.

**Revendications**

**1.** Procédé d'établissement d'un embryon non humain comprenant :

a) l'insertion d'un gène exogène d'intérêt dans un vecteur approprié portant des séquences homologues à un site cible, afin de construire un vecteur ciblé recombinant ;
b) la transfection de cellules ES d'un animal non humain en utilisant ledit vecteur ciblé de l'étape a) et le criblage des cellules ES ciblées qui ont intégré le gène exogène dans un site spécifique ;
c) l'injection des cellules ES ciblées obtenues à partir de l'étape b) dans les blastocystes dudit animal non humain et la culture des blastocystes in vitro de façon que des embryons contenant les cellules ES visées soient générés, et

dans lequel ledit gène exogène d'intérêt est un gène HBV ou HCV et ledit gène exogène d'intérêt est inséré dans le locus p21 dudit animal non humain.

**2.** Procédé selon la revendication 1, dans lequel ledit gène exogène d'intérêt est un gène HBV.

**3.** Procédé selon la revendication 2, dans lequel ledit gène exogène d'intérêt est un gène HBsAg ou un gène HBV X.

**4.** Procédé selon la revendication 1, dans lequel ledit vecteur ciblé comprend un gène exogène d'intérêt, des séquences homologues d'un site cible, des marqueurs de sélection positive et négative.

**5.** Procédé selon la revendication 1, dans lequel ledit animal non humain est une souris.

**6.** Procédé selon la revendication 5, dans lequel ledit gène exogène d'intérêt est inséré dans le deuxième exon du locus p21 de souris.

**7.** Cellule de souris isolée, **caractérisée en ce que** le gène exogène d'intérêt est inséré dans le locus p21 du génome cellulaire et dans laquelle ledit gène exogène d'intérêt est un gène HBV ou HCV.

**8.** Cellule selon la revendication 7, dans laquelle le gène exogène d'intérêt est un gène HBV.

**9.** Cellule selon les revendications 7-8 qui est une cellule souche d'embryon ou une cellule somatique avec le même génotype.

**10.** Cellule selon les revendications 8 ou 9, où ledit gène exogène visé est un gène HBsAg ou un gène HBV X.

**11.** Embryon non humain transgénique obtenu selon le procédé de la revendication 1.

**12.** Souris transgénique qui est **caractérisée par** le chromosome de ladite souris ayant un gène exogène d'intérêt intégré de manière site spécifique par un procédé d'insertion (« knock-in »), dans laquelle ledit gène exogène d'intérêt est un gène HBV, ledit gène exogène d'intérêt étant inséré de manière site spécifique dans le locus p21 de ladite souris.

**13.** Souris transgénique selon la revendication 12, dans laquelle ledit gène exogène d'intérêt est un gène HBsAg ou HBV X.

**14.** Souris transgénique qui est **caractérisée par** un gène HBsAg inséré de manière site spécifique dans le locus p21 de ladite souris, ledit gène exogène d'intérêt étant transmis à la descendance et exprimé de façon stable, ladite souris progressant normalement et développant un carcinome hépatocellulaire après un certain temps.

**15.** Utilisation de la souris transgénique selon l'une quelconque des revendications 12-14 dans la recherche sur la pathogenèse du carcinome hépatocellulaire, son diagnostic précoce et la thérapie.

16. Utilisation de la souris transgénique selon l'une quelconque des revendications 12-14 pour le criblage de médicaments anticancéreux.

**Patentansprüche**

1. Verfahren zum Bilden eines nicht-menschlichen Embryos umfassend:

   a) Einführen eines exogenen Gens von Interesse in einen geeigneten, zu einer Zielstelle homologe Sequenzen tragenden Vektor, um einen rekombinanten zielgerichteten Vektor herzustellen;
   b) Transfizieren von ES-Zellen eines untersuchten nicht-menschlichen Tieres unter Verwendung des zielgerichteten Vektors aus Schritt a) und Überprüfen von Ziel-ES-Zellen, welche das exogene Gen an einer spezifischen Stelle integriert haben;
   c) Injizieren der in Schritt b) erhaltenen Ziel-ES-Zellen in Blastulae der nicht-menschlichen Tiere sowie Kultivieren der Blastulae *in vitro,* so dass Embryonen enthaltend die Ziel-ES-Zellen gebildet werden,

   wobei das exogene Gen von Interesse ein HBV- oder ein HCV-Gen ist und das exogene Gen von Interesse in den p21-Lokus des nicht-menschlichen Tieres eingefügt wird.

2. Verfahren nach Anspruch 1, wobei das exogene Gen von Interesse ein HBV-Gen ist.

3. Verfahren nach Anspruch 2, wobei das exogene Gen von Interesse ein HBsAg-Gen oder ein HBV X-Gen ist.

4. Verfahren nach Anspruch 1, wobei der zielgerichtete Vektor ein exogenes Gen von Interesse, zu einer Zielstelle homologe Sequenzen, positive und negative Selektionsmarker umfasst.

5. Verfahren nach Anspruch 1, wobei das nicht-menschliche Tier eine Maus ist.

6. Verfahren nach Anspruch 5, wobei das exogene Gen von Interesse in das zweite Exon des p21-Lokus der Maus eingefügt wird.

7. Isolierte Mauszelle, **dadurch gekennzeichnet, dass** ein exogenes Gen von Interesse in den p21-Lokus des Zellgenoms eingefügt ist, und, wobei das exogene Gen von Interesse ein HBV- oder ein HCV-Gen ist.

8. Zelle nach Anspruch 7, wobei das exogene Gen von Interesse ein HBV-Gen ist.

9. Zelle nach Anspruch 7 oder 8, welche eine Embryonenstammzelle oder eine somatische Zelle mit demselben Genotyp ist.

10. Zelle nach Anspruch 8 oder 9, wobei das exogene Gen von Interesse ein HBsAg-Gen oder ein HBV X-Gen ist.

11. Transgenes nicht-menschliches Embryo erhalten durch das Verfahren nach Anspruch 1.

12. Transgene Maus, welche **dadurch gekennzeichnet ist, dass** in das Chromosom der Maus ortspezifisch ein exogenes Gen von Interesse durch das Gen knock-in-Verfahren integriert ist, wobei das exogene Gen von Interesse ein HBV-Gen ist und das exogene Gen von Interesse ortspezifisch in den p21-Lokus der Maus eingefügt ist.

13. Transgene Maus nach Anspruch 12, wobei das exogene Gen von Interesse ein HBsAg-Gen oder ein HBV X-Gen ist.

14. Transgene Maus, welche **gekennzeichnet ist durch** ein HBsAg-Gen, welches ortspezifisch in den p21-Lokus der Maus eingefügt ist, wobei das exogene Gen von Interesse stabil vererbt und exprimiert wird, sich die Maus normal entwickelt und nach einer gewissen Zeit Leberzellkarzinome ausbildet.

15. Verwendung der transgenen Maus nach einem der Ansprüche 12 bis 14 zum Untersuchen der Pathogenese von Leberzellkarzinomen, deren Frühstadiumdiagnose und Therapie.

16. Verwendung einer transgenen Maus nach einem der Ansprüche 12 bis 14 zum Screenen von Antikrebsmedikamenten.

Figure 1a

Figure 1b

Figure 1c

Figure 1d

Figure 2a

Figure 2b

Figure 2c

Figure 2d

Figure 3

(a)

(b)

Figure 4

# Genotype identification of transgenic mice containing HBsAg by PCR

Figure 5

## Genotype identification of transgenic mice containing HBsAg by Southern Blot

1 2 3 4 5 6

23kb

9kb
8kb

7.7kb

1、Wt(EcoR I)    2、S/+ (EcoR I)    3、S/S (EcoR I)

4、Wt(Bgl II)    5、S/+(Bgl II)    6、S/S (Bgl II)

Figure 6

# The transcription of HBsAg gene in transgenic mice

1   2   3   4   5   6   7   8   9

1.wild type     2.S/+ spleen     3. S/+ liver

4. S/+ kidney     5.S/+ spermatophore     6.S/+ colon

7.S/+ heart     8.S/S liver     9. S/S liver

EP 1 362 510 B1

Figure 7

## Histopathologic analysis of transgenic mouse

WT S/+

10× 10×

EP 1 362 510 B1

Figure 8

The abnormal proliferation of liver cells in
transgenic mouse

WT        S/+

20×       20×

Figure 9

The elevation of serum α -fetal protein in transgenic mouse

Figure 10a

## The screening of differential genes(one)

1  2  3  4

Est 13

Est 20

Est 38

28S

18S

1、Wt (2m)    2、S/+(2m)    3、Wt(15m)    4、S/+(15m)

Figure 10b

# The screening of differential genes(two)

1  2  3  4  5  6

28S

18S

Aldolase A

EST

H3 histone

Gene A

1.S/S(3 months)

2.S/S (3 months)

3.wild type (3 months)

4.wild type (15 months)

5.S/+ (15 months)

6.tumor (15 months)

Figure 10c

# The screening of differential genes(three)

| | | | | | |
|---|---|---|---|---|---|
| 1 | 2 | 3 | 4 | 5 | 6 |

28S

18S

Ribosomal
protein L8

TNF-alfa
related

Gene B

Ptb

1.S/S(3 months)

2.S/S (3 months)

3.wild type (3 months)

4.wild type (15 months)

5.S/+ (15 months)

6.tumor (15 months)

Figure 10d

# The screening of differential genes(four)

1 2 3 4 5 6 7 8 9 10 11 12 13 14 15 16 17 18 19 20

Est 15

Est 10

Est 14

28S

18S

1. Wt(1d)       2. Wt(2m)      3. S/+(2m)      4. S/S(2m)

5. Wt(4m))     6. S/+(4m)     7. S/S(4m)      8. Wt(5m)

9. S/+(5m)     10. S/S(5m)    11. Wt(12m)     12. S/+(12m)

13. S/S (12m)  14. Wt(15m)    15. S/+(15m)    16. Wt(18m)

17. S/+(18m)   18. Wt(20m)    19. S/+(20m)    20. S/S (20m)

Figure 11

# Two-dimensional(2-D) electrophoresis of proteins

liver proteins in
p21$^{HBsAg/HBsAg}$ mouse

liver proteins in
wild type mouse